# EUROPÄISCHE PATENTSCHRIFT

(11) **EP 2 773 721 B1**
(45) Veröffentlichungstag und Bekanntmachung des Hinweises auf die Patenterteilung: **25.11.2015**
(21) Anmeldenummer: 12775140.2
(22) Anmeldetag: 11.10.2012
(51) Int. Cl.: C09K 11/06, H05B 33/14, C07D 471/16, C07D 471/06, C07D 401/14, C07D 405/10, C07D 221/18, C07D 221/20, C07D 401/04, C07D 401/10, C07D 405/04, C07D 413/10, C07D 405/14, C07D 221/10, C07D 417/04, C07D 471/14, C07D 491/06, C07D 491/22, C07D 495/04, C07D 495/06

(54) **ORGANISCHE ELEKTROLUMINESZENZVORRICHTUNG**
ORGANIC ELECTROLUMINESCENT DEVICE
DISPOSITIF ÉLECTROLUMINESCENT ORGANIQUE

(30) Priorität: 01.11.2011 EP 11008708
(43) Veröffentlichungstag der Anmeldung: 10.09.2014
(73) Patentinhaber: Merck Patent GmbH, 64293 Darmstadt (DE)
(72) Erfinder: PARHAM, Amir Hossain, 65929 Frankfurt am Main (DE); PFLUMM, Christof, 64291 Darmstadt (DE); JATSCH, Anja, 60489 Frankfurt am Main (DE); EBERLE, Thomas, 76829 Landau (DE); STOESSEL, Philipp, 60487 Frankfurt am Main (DE)
(86) Internationale Anmeldenummer: PCT/EP2012/004255
(87) Internationale Veröffentlichungsnummer: WO 2013/064206

(56) Entgegenhaltungen:
- EP-A1- 2 357 177
- EP-A2- 0 761 669
- DE-A1-102010 012 738
- GR-T3- 3 035 253
- JP-A- 7 244 392
- JP-A- 11 514 347
- US-A1- 2007 265 296
- TIMOTHY A. GRESE ET AL: "Photochemical Synthesis of N -Arylbenzophenanthridine Selective Estrogen Receptor Modulators (SERMs)", JOURNAL OF MEDICINAL CHEMISTRY, Bd. 44, Nr. 17, 1. August 2001 (2001-08-01), Seiten 2857-2860, XP055049263, ISSN: 0022-2623, DOI: 10.1021/jm0101601
- ATTILA DEMETER ET AL: "Dual Fluorescence and Intramolecular Charge Transfer with N -Phenylphenanthridinones", THE JOURNAL OF PHYSICAL CHEMISTRY A, Bd. 105, Nr. 19, 21. April 2001 (2001-04-21), Seiten 4611-4621, XP055049265, ISSN: 1089-5639, DOI: 10.1021/jp004555s
- LUDOVIC DONATI ET AL: "Solvent/Base Effects in the Selective Domino Synthesis of Phenanthridinones That Involves High-Valent Palladium Species: Experimental and Theoretical Studies", CHEMISTRY - A EUROPEAN JOURNAL, Bd. 17, Nr. 45, 27. September 2011 (2011-09-27), Seiten 12809-12819, XP055049259, ISSN: 0947-6539, DOI: 10.1002/chem.201101354
- REN WANG ET AL: "One-pot synthesis of benzo[f]quinolin-3-ones and benzo[a]phenanthridein-5-ones by the photoanuulation of 6-chloropyridin-2-ones and 3-chloroisoquinolin-1-ones to phenylacetylene", ORGANIC & BIOMOLECULAR CHEMISTRY, Bd. 9, Nr. 16, 1. Januar 2011 (2011-01-01) , Seite 5802, XP055049260, ISSN: 1477-0520, DOI: 10.1039/c1ob05082f
- LI B ET AL: "One-pot synthesis of benzo[a]phenanthridin-5-ones by photoinduced cycloaddition of 3-chloroisoquinolin-1-ones with styrenes", TETRAHEDRON LETTERS, ELSEVIER, AMSTERDAM, NL, Bd. 51, Nr. 29, 21. Juli 2010 (2010-07-21) , Seiten 3748-3751, XP027089265, ISSN: 0040-4039 [gefunden am 2010-06-15]
- ATTILA DEMETER ET AL: "Dual luminescence properties of differently benzo-fused N-phenylphenanthridinonesDedicated to Professor Jean Kossanyi on the occasion of his 70th birthday.", PHOTOCHEMICAL & PHOTOBIOLOGICAL SCIENCES, Bd. 2, Nr. 3, 1. Januar 2003 (2003-01-01), Seite 273, XP055049262, ISSN: 1474-905X, DOI: 10.1039/b210592f
- GOMEZ-LOR BERTA ET AL: "Synthesis of a Triaza Analogue of Crushed-Fullerene by Intramolecular Palladium-Catalyzed Arylation", ORGANIC LETTERS, AMERICAN CHEMICAL SOCIETY, US, Bd. 6, Nr. 17, 1. Januar 2004 (2004-01-01) , Seiten 2993-2996, XP002543593, ISSN: 1523-7060, DOI: 10.1021/OL048760S [gefunden am 2004-07-24]
- HEY D H ET AL: "INTERNUCLEAR CYCLISATION. PART XIII. THE DECOMPOSITION OF DIAZONIUM SALTS PREPARED FROM N-O-AMINOBENZOYLDIPHENYLAMINES. A NEW MOLECULAR REARRANGEMENT", JOURNAL OF THE CHEMICAL SOCIETY, CHEMICAL SOCIETY, LETCHWORTH; GB, 1. Januar 1959 (1959-01-01), Seiten 1563-1572, XP008076007, ISSN: 0368-1769

## Beschreibung

Die vorliegende Erfindung betrifft elektronische Vorrichtungen, insbesondere organische Elektrolumineszenzvorrichtungen, Materialien für die Verwendung in elektronischen Vorrichtungen, insbesondere in organischen Elektrolumineszenzvorrichtungen, sowie ein Verfahren zur Herstellung dieser Materialien.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden hierbei zunehmend metallorganische Komplexe eingesetzt, die Phosphoreszenz statt Fluoreszenz zeigen. Aus quantenmechanischen Gründen ist unter Verwendung metallorganischer Verbindungen als Phosphoreszenzemitter eine bis zu vierfache Quanten- und Leistungseffizienz möglich. Generell gibt es bei OLEDs, insbesondere auch bei OLEDs, die Triplettemission (Phosphoreszenz) zeigen, immer noch Verbesserungsbedarf, beispielsweise im Hinblick auf Effizienz, Betriebsspannung und Lebensdauer. Dies gilt insbesondere für OLEDs, welche im kürzerwelligen Bereich, beispielsweise grün, emittieren.

Die Eigenschaften von phosphoreszierenden OLEDs werden nicht nur von den eingesetzten Triplettemittern bestimmt. Hier sind insbesondere auch die anderen verwendeten Materialien, wie Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können somit auch zu deutlichen Verbesserungen der OLED-Eigenschaften führen. Auch für fluoreszierende OLEDs gibt es bei diesen Materialien noch Verbesserungsbedarf.

Gemäß dem Stand der Technik werden unter anderem Ketone (z. B. gemäß WO 2004/093207 oder WO 2010/006680) als Matrixmaterialien für phosphoreszierende Emitter verwendet. Allerdings besteht bei Verwendung dieser Matrixmaterialien ebenso wie bei anderen Matrixmaterialien noch Verbesserungsbedarf, insbesondere in Bezug auf die Effizienz und die Lebensdauer der Vorrichtung.

Gemäß dem Stand der Technik werden weiterhin Carbazolderivate, z. B. gemäß WO 2005/039246, US 2005/0069729, JP 2004/288381, EP 1205527 oder WO 2008/086851, und Indolocarbazolderivate, z. B. gemäß WO 2007/063754 oder WO 2008/056746, als Matrixmaterialien für phosphoreszierende Emitter in organischen Elektrolumineszenzvorrichtungen eingesetzt. Diese sind teilweise elektrochemisch nicht stabil, was zu Nebenreaktionen in der organischen Elektrolumineszenzvorrichtung führt. Hier sind weitere Verbesserungen wünschenswert, ebenso wie in Bezug auf die Effizienz, die Lebensdauer und die thermische Stabilität der Materialien.

Aufgabe der vorliegenden Erfindung ist die Bereitstellung von Verbindungen, welche sich für den Einsatz in einer fluoreszierenden oder phosphoreszierenden OLED, insbesondere einer phosphoreszierenden OLED, eignen, beispielsweise als Matrixmaterial oder als Lochtransport-/ Elektronenblockiermaterial bzw. Exzitonenblockiermaterial oder als Elektronentransport- bzw. Lochblockiermaterial. Insbesondere ist es die Aufgabe der vorliegenden Erfindung, Matrixmaterialien bereitzustellen, welche sich für grün, rot und gegebenenfalls auch blau phosphoreszierende OLEDs eignen.

Überraschend wurde gefunden, dass die unten näher beschriebenen Verbindungen diese Aufgabe lösen und zu Verbesserungen der organischen Elektrolumineszenzvorrichtung führen, insbesondere hinsichtlich der Lebensdauer, der Effizienz und/oder der Betriebsspannung. Dies gilt insbesondere für rot und grün phosphoreszierende Elektrolumineszenzvorrichtungen, vor allem bei Einsatz der erfindungsgemäßen Verbindungen als Matrixmaterial. Die Materialien zeichnen sich weiterhin durch hohe Oxidationsstabilität in Lösung sowie durch eine hohe Temperaturstabilität aus. Elektronische Vorrichtungen, insbesondere organische Elektrolumineszenzvorrichtungen, welche derartige Verbindungen enthalten, sind daher der Gegenstand der vorliegenden Erfindung.

Gegenstand der vorliegenden Erfindung ist eine elektronische Vorrichtung enthaltend eine Verbindung gemäß Formel (1), 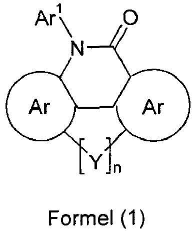 wobei für die verwendeten Symbole und Indizes gilt:
- Ar: ist bei jedem Auftreten gleich oder verschieden eine Aryl- oder Heteroarylgruppe mit 5 bis 13 aromatischen Ringatomen, welche durch einen oder mehrere Reste R substituiert sein kann;
- Ar¹: ist ein aromatisches oder heteroaromatisches Ringsystem mit 5 bis 24 aromatischen Ringatomen, welches durch einen oder mehrere Reste R substituiert sein kann und welches keine Aryl- oder Heteroarylgruppen mit mehr als zwei direkt aneinander kondensierten aromatischen Sechsringen aufweist;
- Y: ist -C(=O)-N(Ar¹)-, -C(=O)-O-, -CR¹=CR¹-, -CR¹=N-, C(R¹)₂, NR¹, O, S, C(=O), C(=S), C(=NR¹), C(=C(R¹)₂), Si(R¹)₂, BR¹, PR¹, P(=O)R¹, SO oder SO₂;
- R, R¹: ist bei jedem Auftreten gleich oder verschieden ausgewählt aus der Gruppe bestehend aus H, D, F, Cl, Br, I, CN, NO₂, N(Ar²)₂, N(R²)₂, C(=O)Ar², C(=O)R², P(=O)(Ar²)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R² substituiert sein kann, wobei eine oder mehrere nicht-benachbarte CH₂-Gruppen durch R²C=CR², C≡C, Si(R²)₂, C=O, C=S, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R² substituiert sein kann, einer Aryloxy- oder Heteroaryloxygruppe mit 5 bis 60 aromatischen Ringatomen, die mit einem oder mehreren Resten R² substituiert sein kann, oder einer Kombination dieser Systeme, wobei optional zwei oder mehr benachbarte Substituenten R bzw. zwei oder mehr benachbarte Substituenten R¹ ein monocyclisches oder polycyclisches, aliphatisches, aromatisches oder heteroaromatisches Ringsystem bilden können, das mit einem oder mehreren Resten R² substituiert sein kann und welches keine Aryl- oder Heteroarylgruppen mit mehr als zwei direkt aneinander kondensierten aromatischen Sechsringen aufweist; weiterhin kann auch ein Rest R an Ar¹ mit einem Rest R an Ar ein aliphatisches Ringsystem bilden; mit der Maßgabe, dass durch Ringbildung der Reste R bzw. R¹ keine Aryl- bzw. Heteroarylgruppe mit mehr als zwei direkt aneinander kondensierten Arylgruppen entsteht;
- Ar²: ist bei jedem Auftreten gleich oder verschieden ein aromatisches oder heteroaromatisches Ringsystem mit 5-30 aromatischen Ringatomen, das mit einem oder mehreren nicht-aromatischen Resten R² substituiert sein kann und welches keine Aryl- oder Heteroarylgruppen mit mehr als zwei direkt aneinander kondensierten aromatischen Sechsringen aufweist; dabei können zwei Reste Ar², welche an dasselbe N-Atom oder P-Atom binden, auch durch eine Einfachbindung oder eine Brücke, ausgewählt aus N(R²), C(R²)₂ oder O, miteinander verbrückt sein;
- R²: ist ausgewählt aus der Gruppe bestehend aus H, D, F, CN, einem aliphatischem Kohlenwasserstoffrest mit 1 bis 20 C-Atomen, einem aromatischem oder heteroaromatischem Ringsystem mit 5 bis 30 aromatischen Ringatomen, in dem ein oder mehrere H-Atome durch D, F, Cl, Br, I oder CN ersetzt sein können, wobei zwei oder mehr benachbarte Substituenten R² miteinander ein mono- oder polycyclisches, aliphatisches, aromatisches oder heteroaromatisches Ringsystem bilden können;
- n: ist bei jedem Auftreten gleich oder verschieden 0 oder 1, wobei n = 0 bedeutet, dass keine Gruppe Y vorhanden ist und statt dessen in den Positionen an Ar, in denen in Formel (1) Y gebunden ist, ein Substituent R gebunden ist oder ein Heteroatom der Gruppe Ar vorhanden ist;
mit der Maßgabe, dass die Reste an Ar oder Ar¹ keine Lactamgruppe enthalten.

Eine elektronische Vorrichtung im Sinne der vorliegenden Erfindung ist eine Vorrichtung, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen farbstoffsensibilisierten Solarzellen (O-DSSC), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs) und besonders bevorzugt phosphoreszierenden OLEDs.

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 80 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind.

Unter einer Aryl- oder Heteroarylgruppe mit nicht mehr als zwei direkt aneinander kondensierten aromatischen Sechsringen werden einfache Aryl- oder Heteroarylgruppen, wie zum Beispiel Benzol oder Pyridin, oder Aryl- oder Heteroarylgruppen mit genau zwei aneinander kondensierten aromatischen Sechsringen, wie zum Beispiel Naphthalin oder Chinolin verstanden. Weiterhin werden hierunter Heteroarylgruppen verstanden, in denen aromatische Fünfringe und Sechsringe, aber nicht aromatische Sechsringe direkt aneinander ankondensiert sind, wie zum Beispiel Carbazol, Dibenzofuran, Dibenzothiophen oder Benzimidazol.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cyclo-heptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 aromatischen Ringatomen, welches noch jeweils mit den oben genannten Resten R² oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Pyren, Benzanthracen, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolo-carbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.

Unter benachbarten Resten R bzw. R¹ im Sinne der vorliegenden Erfindung werden Reste verstanden, die an Kohlenstoffatomen binden, welche direkt aneinander gebunden sind, oder Reste, die an dasselbe Kohlenstoffatom binden.

In einer bevorzugten Ausführungsform der Erfindung ist Ar gleich oder verschieden bei jedem Auftreten ausgewählt aus einer Aryl- oder Heteroarylgruppe mit 6 aromatischen Ringatomen oder einer Heteroarylgruppe mit 5 aromatischen Ringatomen, welches jeweils mit einem oder mehreren Resten R substituiert sein kann. Dabei können zwei Substituenten R auch miteinander ein weiteres Ringsystem bilden. In einer bevorzugten Ausführungsform der Erfindung ist Ar für n = 0 ausgewählt aus den Gruppen der folgenden Formeln (2) bis (5) und für n = 1 aus den Gruppen der folgenden Formeln (6) bis (8), mit der Maßgabe, dass für n = 1 mindestens eine Gruppe Ar für eine Gruppe der Formel (6) steht, 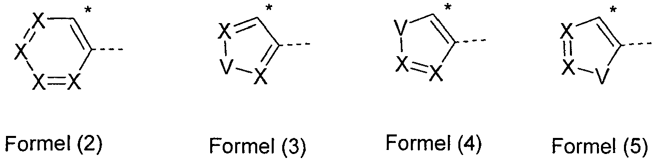 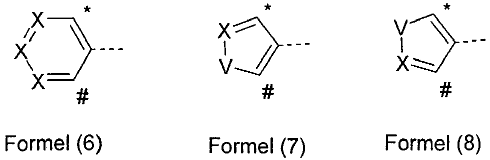 wobei die gestrichelte Bindung die Verknüpfung mit der zweiten Gruppe Ar andeutet, # die Position der Verknüpfung mit Y andeutet, * die Verknüpfung mit dem Stickstoffatom bzw. der Carbonylgruppe des Lactams andeutet und weiterhin gilt:
- X: ist gleich oder verschieden bei jedem Auftreten CR oder N; oder zwei benachbarte Gruppen X in Formel (2) oder Formel (6) stehen für eine Gruppe der folgenden Formel (9) oder (10), 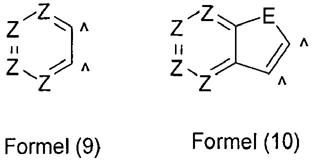 wobei E für NR, CR₂, O oder S steht, Z gleich oder verschieden bei jedem Auftreten für CR oder N steht und ^ die entsprechenden benachbarten Gruppen X in Formel (2) oder Formel (6) andeuten;
- V: ist NR, O oder S.

Besonders bevorzugt ist Ar gleich oder verschieden bei jedem Auftreten ausgewählt aus der Gruppe bestehend aus Benzol, Pyridin, Thiophen, Furan, Pyrrol, Carbazol, Fluoren, Benzofuran, Benzothiophen, Dibenzofuran oder Dibenzothiophen, welches jeweils mit einem oder mehreren Resten R substituiert sein kann. Ganz besonders bevorzugt ist Ar bei jedem Auftreten Benzol, welches durch einen oder mehrere Reste R substituiert sein kann, steht also ganz besonders bevorzugt für eine Gruppe der Formel (2) bzw. Formel (6), in der jeweils alle Gruppen X für CR stehen.

In einer weiteren bevorzugten Ausführungsform der Erfindung stehen beide Gruppen Ar für die gleiche Aryl- oder Heteroarylgruppe. Dabei können die beiden Gruppen Ar gleich oder unterschiedlich substituiert sein.

Bevorzugte Ausführungsfomen der Verbindungen der Formel (1) sind die Verbindungen der folgenden Formeln (11) bis (21), 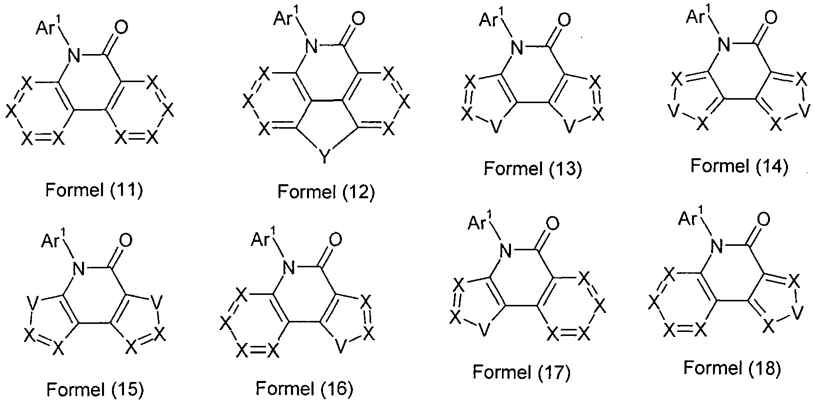 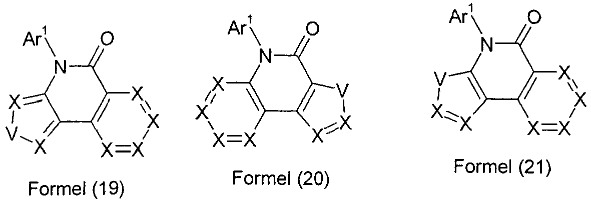 wobei die verwendeten Symbole die oben genannten Bedeutungen aufweisen.

Besonders bevorzugt sind die Verbindungen der oben genannten Formeln (11) und (12).

In einer bevorzugten Ausführungsform der Erfindung steht maximal eine Gruppe X pro Cyclus für N und die anderen Gruppen X stehen gleich oder verschieden bei jedem Auftreten für CR. In einer besonders bevorzugten Ausführungsform der Erfindung stehen alle Gruppen X gleich oder verschieden bei jedem Auftreten für CR.

Besonders bevorzugt sind daher die Verbindungen der folgenden Formeln (11a) und (12a), 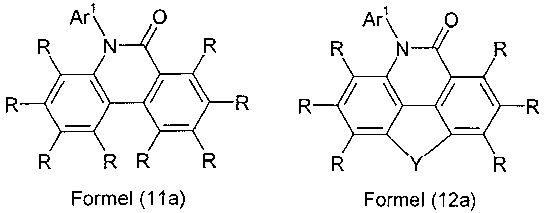 wobei die verwendeten Symbole die oben genannten Bedeutungen aufweisen.

Ganz besonders bevorzugt sind die Verbindungen der folgenden Formeln (11b), (11c), (11d), (12b), (12c) und (12d), 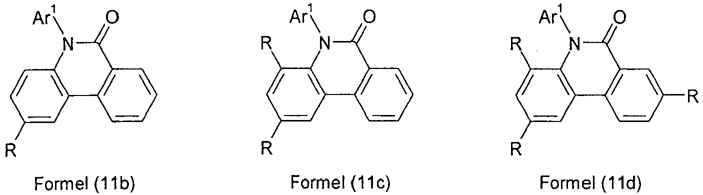 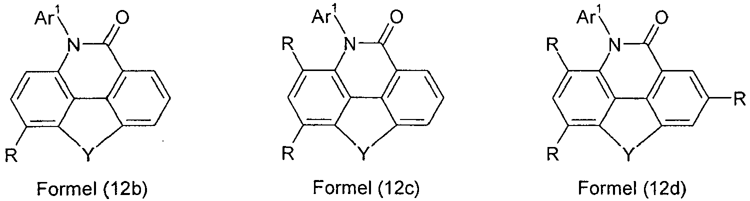 wobei die verwendeten Symbole die oben genannten Bedeutungen aufweisen.

Wie oben bereits beschrieben, stellt Ar¹ ein aromatisches oder heteroaromatisches Ringsystem mit 5 bis 24 aromatischen Ringatomen dar, welches durch einen oder mehrere Reste R substituiert sein kann und welches keine Aryl- oder Heteroarylgruppen mit mehr als zwei direkt aneinander kondensierten aromatischen Sechsringen enthält. Bevorzugt enthält Ar¹ und auch die Reste R, die an Ar¹ gebunden sind, überhaupt keine direkt aneinander kondensierten aromatischen Sechsringe.

Besonders bevorzugte Gruppen Ar¹ sind ausgewählt aus der Gruppe bestehend aus Benzol, ortho-, meta- oder para-Biphenyl, ortho-, meta-, para- oder verzweigtes Terphenyl, lineares oder verzweigtes Quaterphenyl, Fluoren, insbesondere 2-Fluoren, Spirobifluoren, insbesondere 2-Spirobifluoren, Carbazol, insbesondere 2- oder 3-Carbazol, Dibenzothiophen, insbesondere 1-, 2- oder 3-Dibenzothiophen, Dibenzofuran, insbesondere 1-, 2- oder 3-Dibenzofuran, 1,3,5-Triazin, Pyridin, Pyrimidin, Indenocarbazol, verbrücktes Carbazol oder Indolocarbazol oder Kombinationen aus zwei oder drei dieser Gruppen. Dabei können diese Gruppen jeweils auch durch einen oder mehrere Reste R substituiert sein.

In einer bevorzugten Ausführungsform der Erfindung ist der Index n = 0.

In einer weiteren bevorzugten Ausführungsform der Erfindung ist der Index n = 1 und Y ist ausgewählt aus der Gruppe bestehend aus -C(=O)-N(Ar¹)-, -C(=O)-O-, C(=O), C(R¹)₂ oder NR¹, besonders bevorzugt -C(=O)-N(Ar¹)- oder C(R¹)₂. Diese Bevorzugung gilt auch für die oben genannten Verbindungen der Formel (12) bzw. (12a) bis (12d). Dabei kann die Gruppe Y in beiden möglichen Orientierungen gebunden sein. Dies wird im Folgenden schematisch an einer Verbindung mit Y = -C(=O)-N(Ar¹)-veranschaulicht:

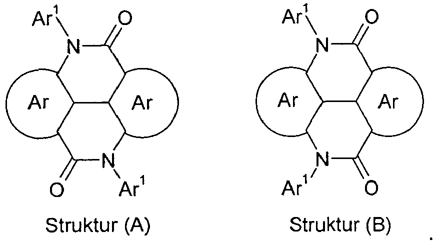

Dabei ist die in in Struktur (A) gezeigte Orientierung, in der an jeder Gruppe Ar jeweils eine Carbonylgruppe und eine Gruppe NAr¹ gebunden ist, die bevorzugte Orientierung.

Wenn die Gruppe Y für eine Gruppe -C(=O)-N(Ar¹)- steht, dann ist die Gruppe Ar¹ bevorzugt ausgewählt aus den oben als bevorzugt genannten Gruppen Ar¹. Besonders bevorzugt sind in diesem Fall die beiden Gruppen Ar¹ im Molekül gleich gewählt.

In einer bevorzugten Ausführungsform der Erfindung ist R in den oben genannten Formeln gleich oder verschieden bei jedem Auftreten ausgewählt aus der Gruppe bestehend aus H, D, F, CN, N(Ar²)₂, C(=O)Ar², einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten R² substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 30 aromatischen Ringatomen, das jeweils mit einem oder mehreren Resten R² substituiert sein kann, oder einer Aryloxy- oder Heteroaryloxygruppe mit 5 bis 30 aromatischen Ringatomen, die mit einem oder mehreren Resten R² substituiert sein kann.

In einer besonders bevorzugten Ausführungsform der Erfindung ist R in den oben genannten Formeln gleich oder verschieden bei jedem Auftreten ausgewählt aus der Gruppe bestehend aus H, einer geradkettigen Alkylgruppe mit 1 bis 10 C-Atomen oder einer verzweigten oder cyclischen Alkylgruppe mit 3 bis 10 C-Atomen, die jeweils mit einem oder mehreren Resten R² substituiert sein kann, oder einem aromatischen oder heteroaromatischen Ringsystem mit 5 bis 18 aromatischen Ringatomen, das jeweils mit einem oder mehreren Resten R² substituiert sein kann.

Insbesondere bevorzugt steht R für ein aromatisches oder heteroaromatisches Ringsystem mit 5 bis 18 aromatischen Ringatomen, das jeweils mit einem oder mehreren Resten R² substituiert sein kann. Wenn R für ein aromatisches oder heteroaromatisches Ringsystem steht, so enthält dieses bevorzugt keine Aryl- oder Heteroarylgruppen, in denen mehr als zwei aromatische Sechsringe direkt aneinander ankondensiert sind, und enthält besonders bevorzugt überhaupt keine Aryl- oder Heteroarylgruppen, in denen aromatische Sechsringe direkt aneinander ankondensiert sind.

Wenn R als Substituent an den Gruppen Ar für ein aromatisches oder heteroaromatisches Ringsystem steht, so ist dieses besonders bevorzugt ausgewählt aus der Gruppe bestehend aus Benzol, ortho-, meta- oder para-Biphenyl, ortho-, meta-, para- oder verzweigtes Terphenyl, lineares oder verzweigtes Quaterphenyl, Fluoren, insbesondere 2-Fluoren, Spirobifluoren, insbesondere 2-Spirobifluoren, Carbazol, insbesondere 2- oder 3-Carbazol oder N-Carbazol, Dibenzothiophen, insbesondere 1-, 2- oder 3-Dibenzothiophen, Dibenzofuran, insbesondere 1-, 2- oder 3-Dibenzofuran, 1,3,5-Triazin, Pyridin, Pyrimidin oder Kombinationen aus zwei oder drei dieser Gruppen. Dabei können diese Gruppen jeweils auch durch einen oder mehrere Reste R² substituiert sein.

Dabei haben für Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

In einer besonders bevorzugten Ausführungsform der Erfindung treten die oben genannten Bevorzugungen gleichzeitig auf.

Beispiele für bevorzugte Verbindungen gemäß den oben aufgeführten Ausführungsformen sind die Verbindungen der folgenden Strukturen (1) bis (201).

| | | |
|---|---|---|
| 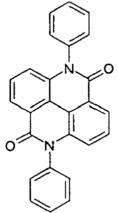 | 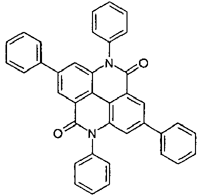 | 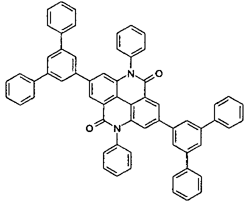 |
| (1) | (2) | (3) |
| 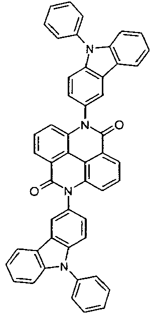 | 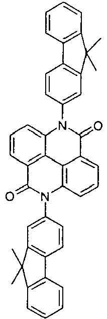 | 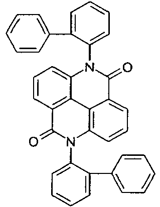 |
| (4) | (5) | (6) |
| 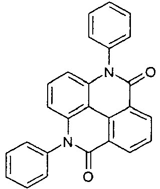 | 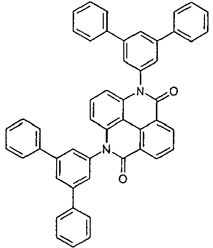 | 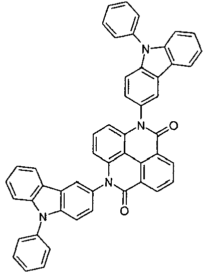 |
| (7) | (8) | (9) |
| 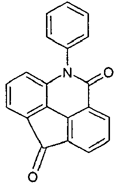 | 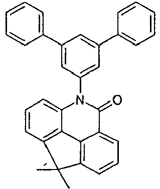 | 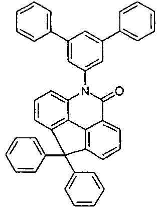 |
| (10) | (11) | (12) |
| 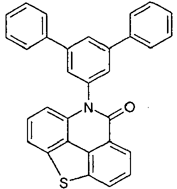 | 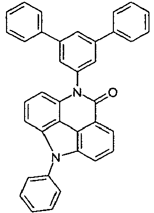 | 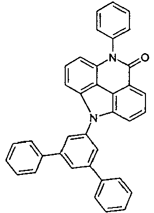 |
| (13) | (14) | (15) |
| 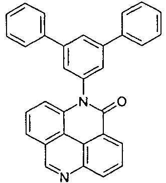 | 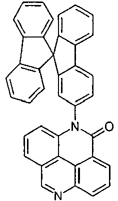 | 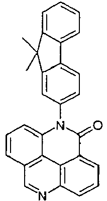 |
| (16) | (17) | (18) |
| 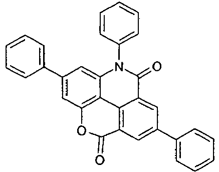 | 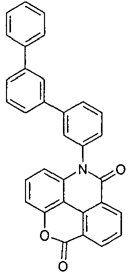 | 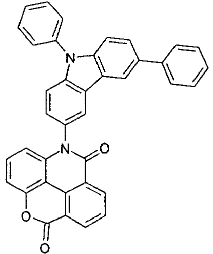 |
| (19) | (20) | (21) |
| 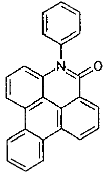 | 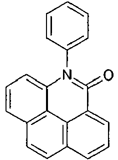 | 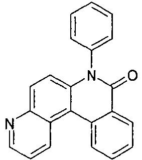 |
| (22) | (23) | (24) |
| 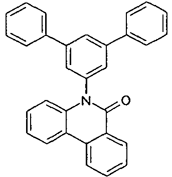 | 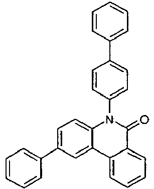 | 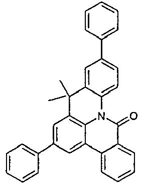 |
| (25) | (26) | (27) |
| 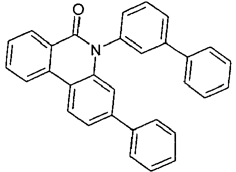 | 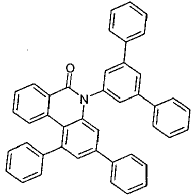 | 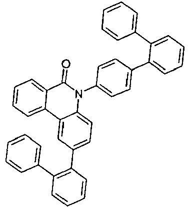 |
| (28) | (29) | (30) |
| 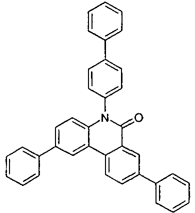 | 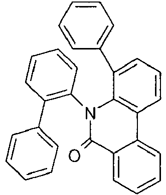 | 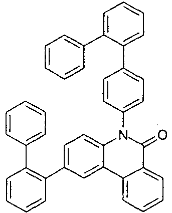 |
| (31) | (32) | (33) |
| 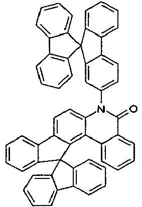 | 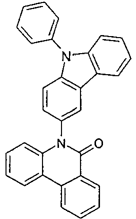 | 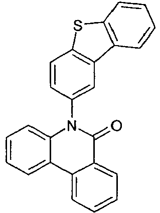 |
| (34) | (35) | (36) |
| 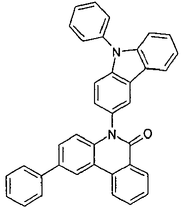 | 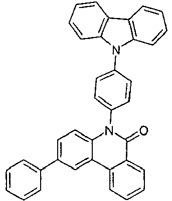 | 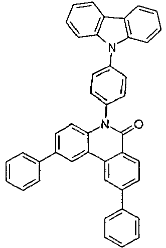 |
| (37) | (38) | (39) |
| 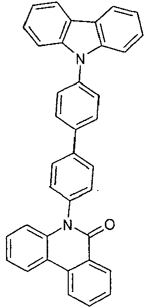 | 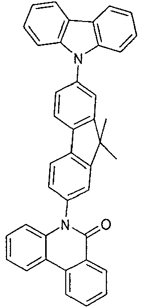 | 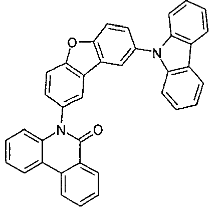 |
| (40) | (41) | (42) |
| 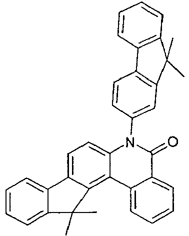 | 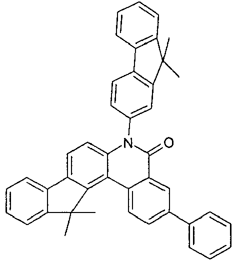 | 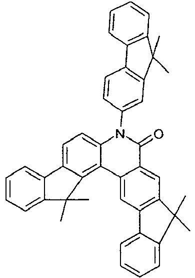 |
| (43) | (44) | (45) |
| 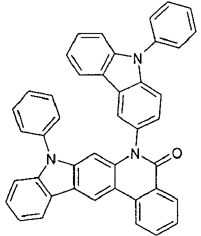 | 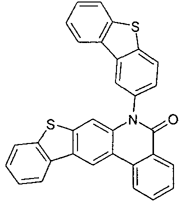 | 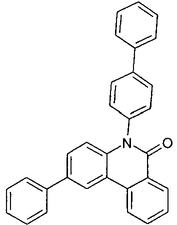 |
| (46) | (47) | (48) |
| 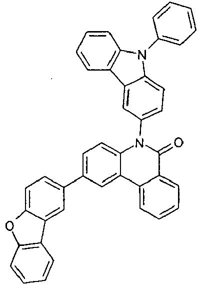 | 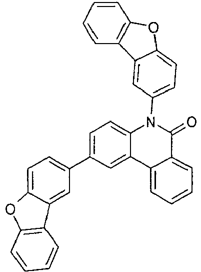 | 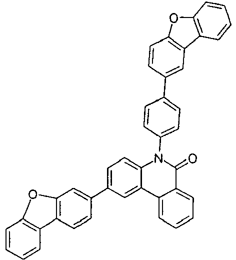 |
| (49) | (50) | (51) |
| 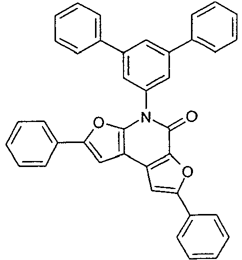 | 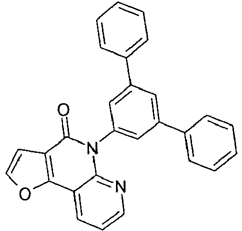 | 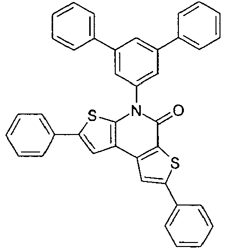 |
| (52) | (53) | (54) |
| 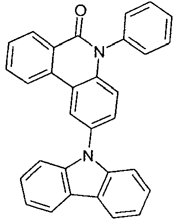 | 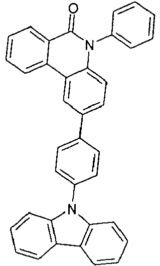 | 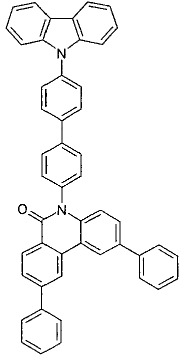 |
| (55) | (56) | (57) |
| 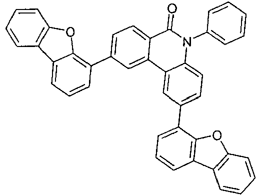 | 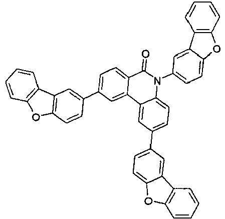 | 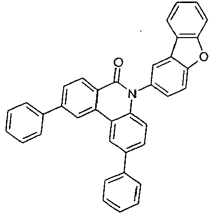 |
| (58) | (59) | (60) |
| 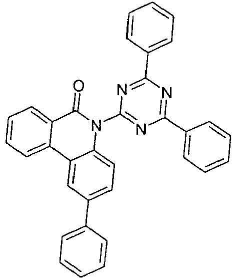 | 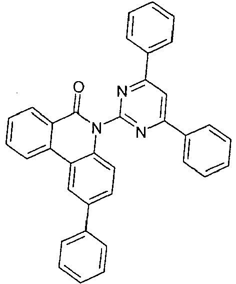 | 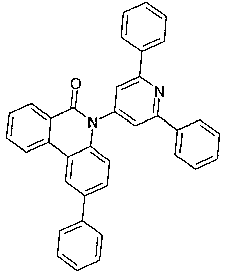 |
| (61) | (62) | (63) |
| 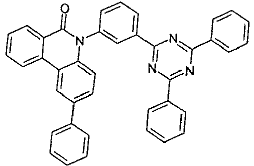 | 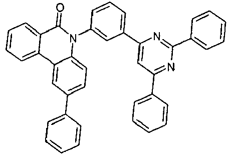 | 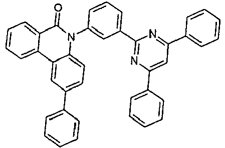 |
| (64) | (65) | (66) |
| 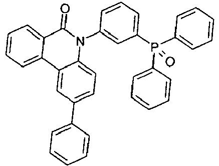 | 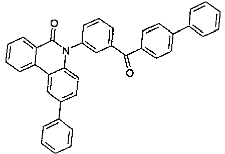 | 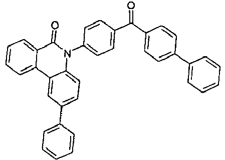 |
| (67) | (68) | (69) |
| 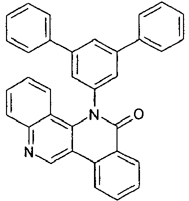 | 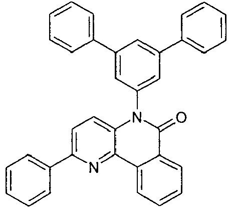 | 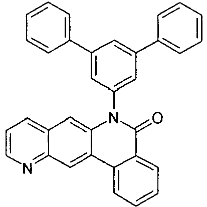 |
| (70) | (71) | (72) |
| 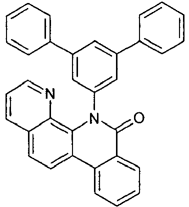 | 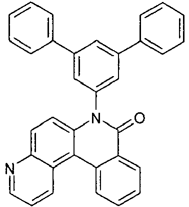 | 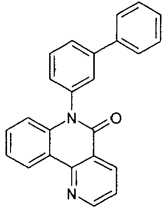 |
| (73) | (74) | (75) |
| 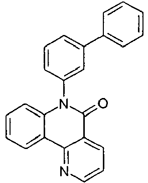 | 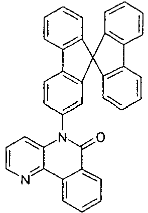 | 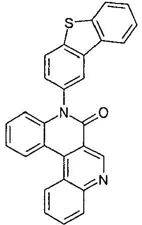 |
| (76) | (77) | (78) |
| 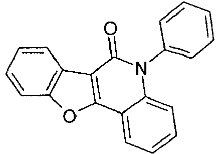 | 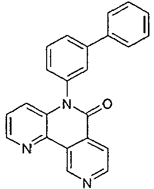 | 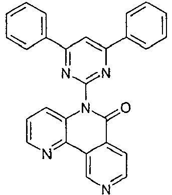 |
| (79) | (80) | (81) |
| 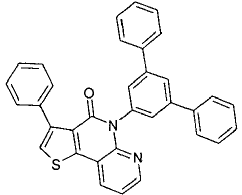 | 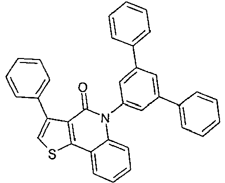 | 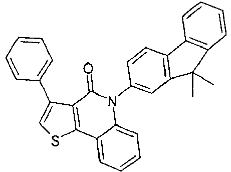 |
| (82) | (83) | (84) |
| 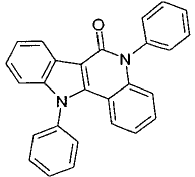 | 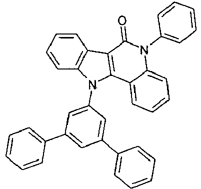 | 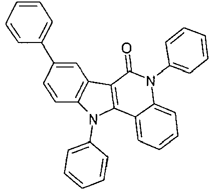 |
| (85) | (86) | (87) |
| 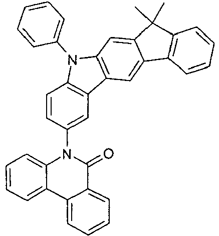 | 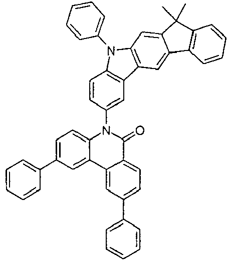 | 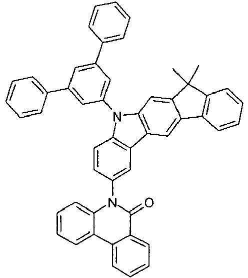 |
| (88) | (89) | (90) |
| 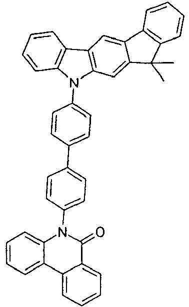 | 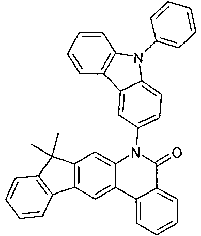 | 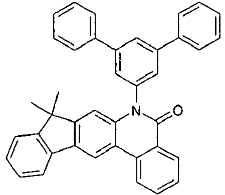 |
| (91) | (92) | (93) |
| 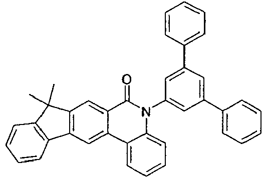 | 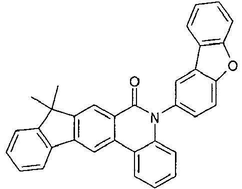 | 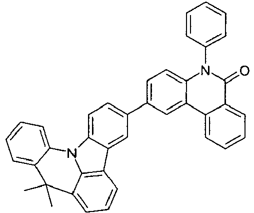 |
| (94) | (95) | (96) |
| 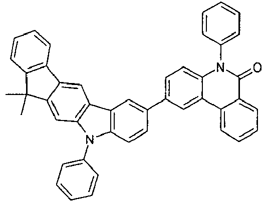 | 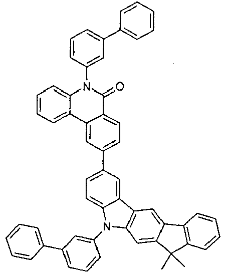 | 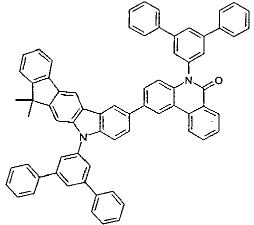 |
| (97) | (98) | (99) |
| 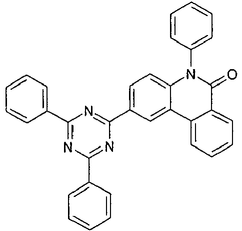 | 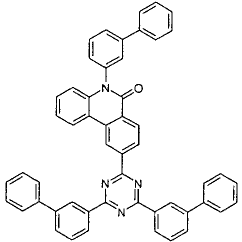 | 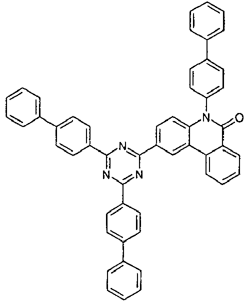 |
| (100) | (101) | (102) |
| 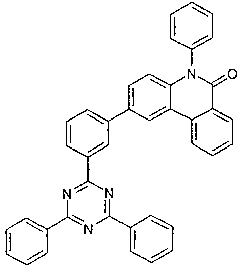 | 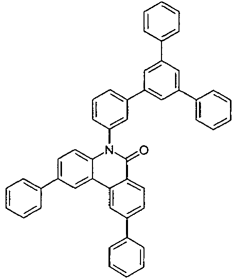 | 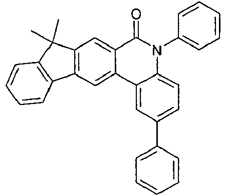 |
| (103) | (104) | (105) |
| 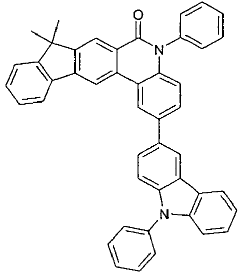 | 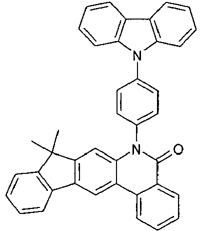 | 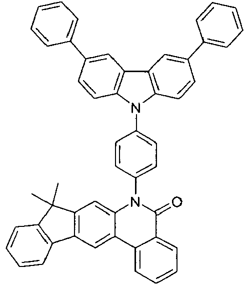 |
| (106) | (107) | (108) |
| 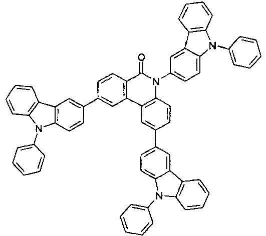 | 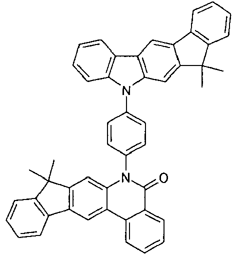 | 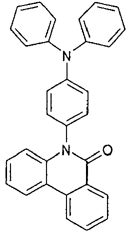 |
| (109) | (110) | (111) |
| 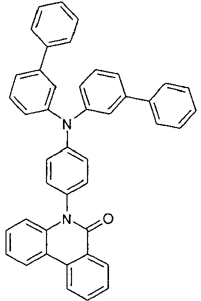 | 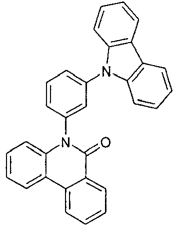 | 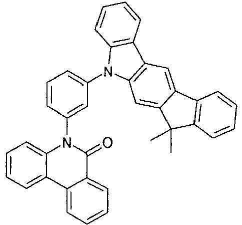 |
| (112) | (113) | (114) |
| 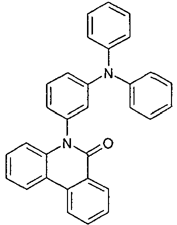 | 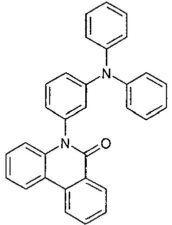 | 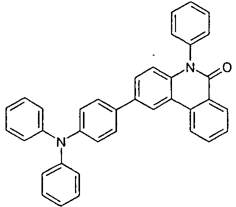 |
| (115) | (116) | (117) |
| 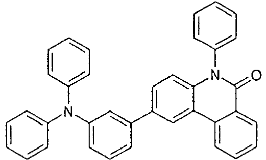 | 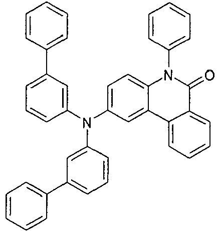 | 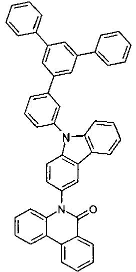 |
| (118) | (119) | (120) |
| 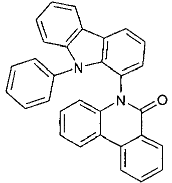 | 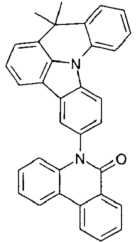 | 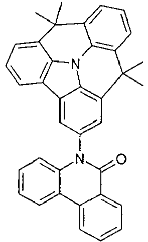 |
| (121) | (122) | (123) |
| 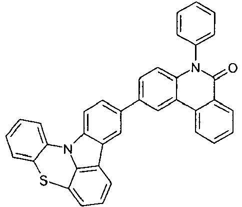 | 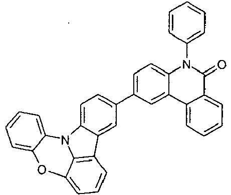 | 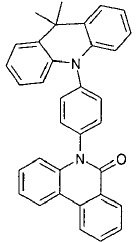 |
| (124) | (125) | (126) |
| 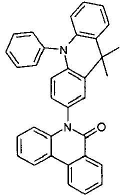 | 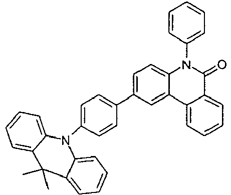 | 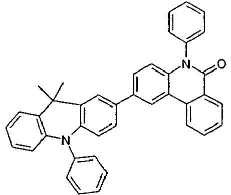 |
| (127) | (128) | (129) |
| 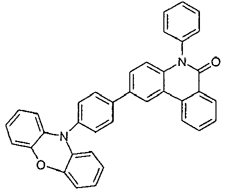 | 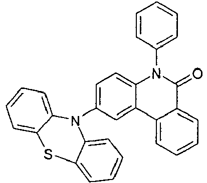 | 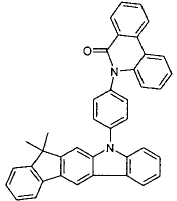 |
| (130) | (131) | (132) |
| 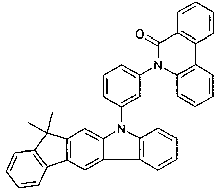 | 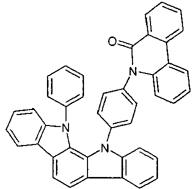 | 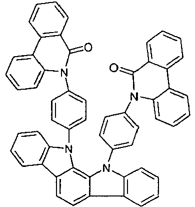 |
| (133) | (134) | (135) |
| 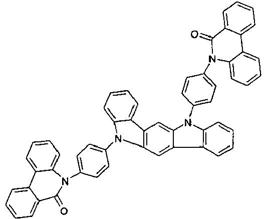 | 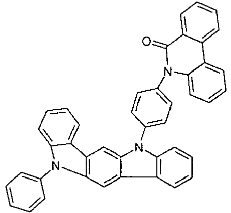 | 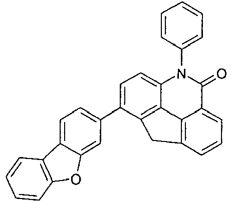 |
| (136) | (137) | (138) |
| 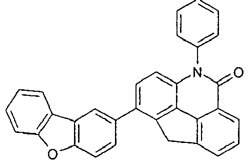 | 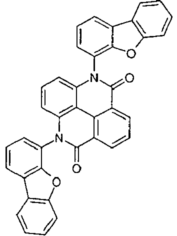 | 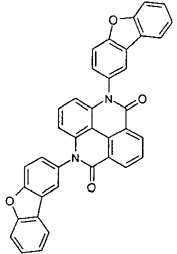 |
| (139) | (140) | (141) |
| 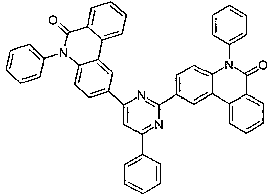 | 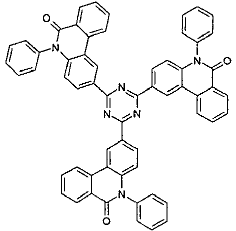 | 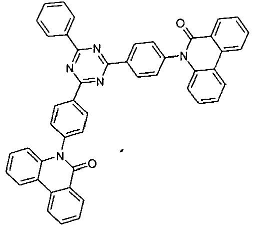 |
| (142) | (143) | (144) |
| 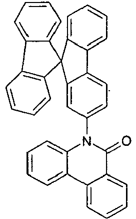 | 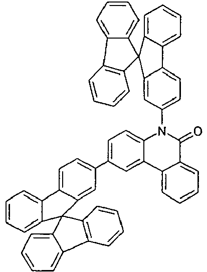 | 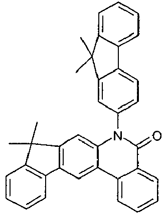 |
| (145) | (146) | (147) |
| 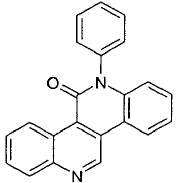 | 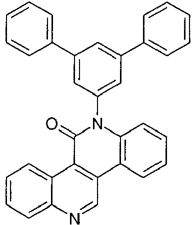 | 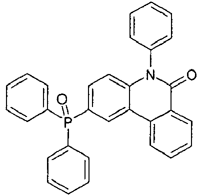 |
| (148) | (149) | (150) |
| 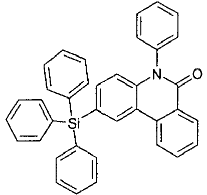 | 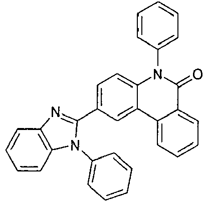 | 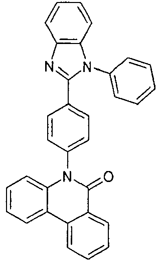 |
| (151) | (152) | (153) |
| 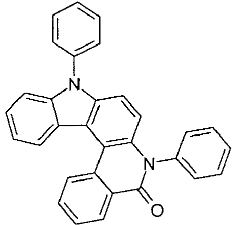 | 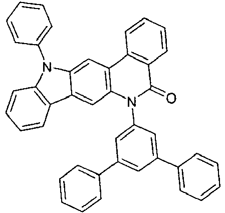 | 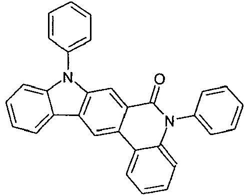 |
| (154) | (155) | (156) |
| 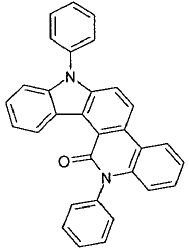 | 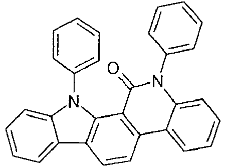 | 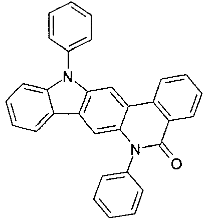 |
| (157) | (158) | (159) |
| 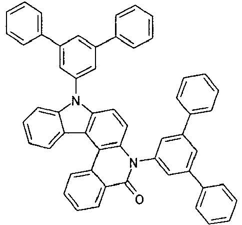 | 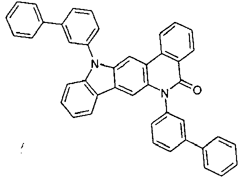 | 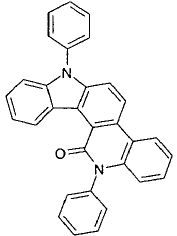 |
| (160) | (161) | (162) |
| 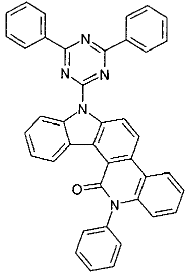 | 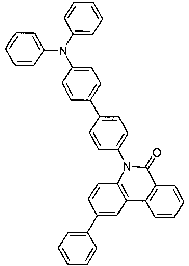 | 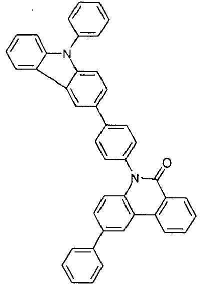 |
| (163) | (164) | (165) |
| 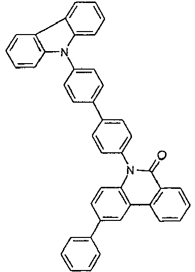 | 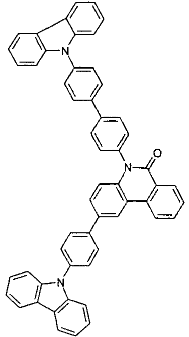 | 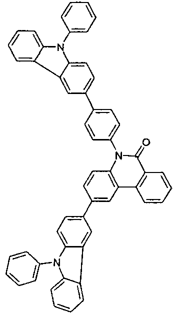 |
| (166) | (167) | (168) |
| 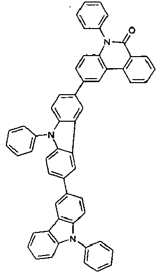 | 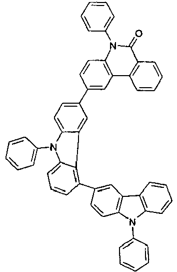 | 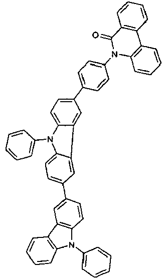 |
| (169) | (170) | (171) |
| 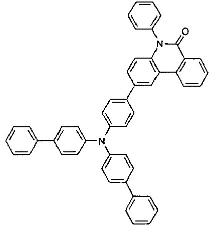 | 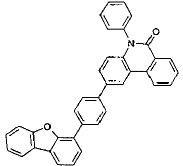 | 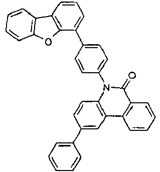 |
| (172) | (173) | (174) |
| 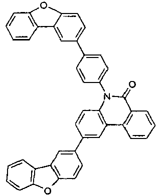 | 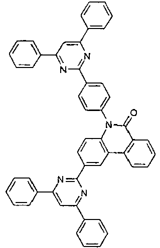 | 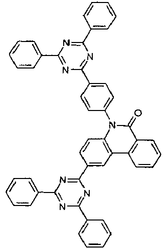 |
| (175) | (176) | (177) |
| 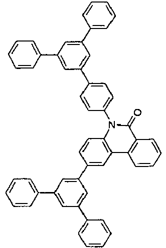 | 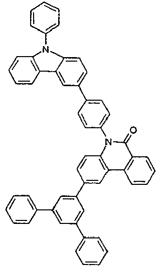 | 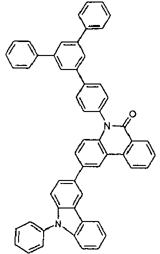 |
| (178) | (179) | (180) |
| 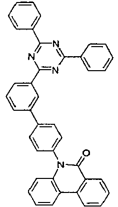 | 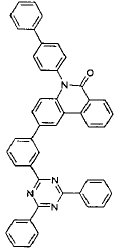 | 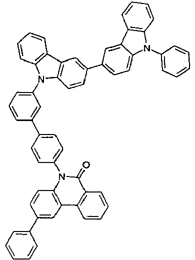 |
| (181) | (182) | (183) |
| 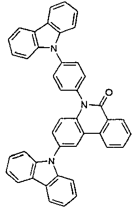 | 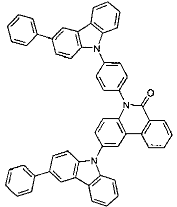 | 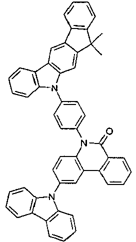 |
| (184) | (185) | (186) |
| 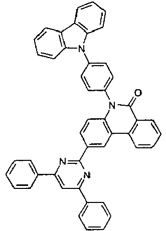 | 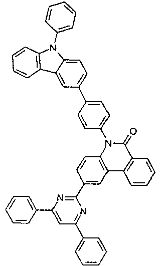 | 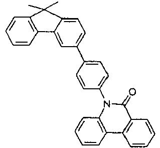 |
| (187) | (188) | (189) |
| 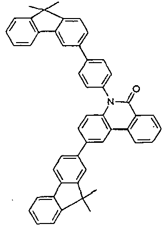 | 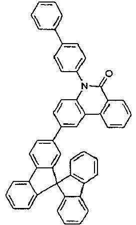 | 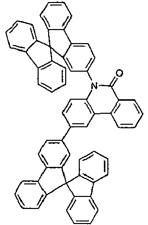 |
| (190) | (191) | (192) |
| 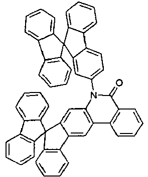 | 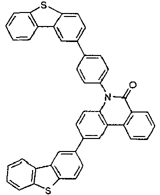 | 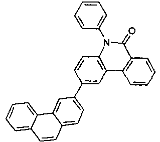 |
| (193) | (194) | (195) |
| 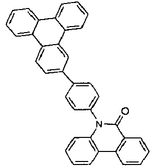 | 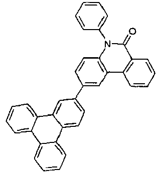 | 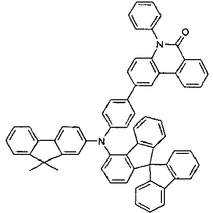 |
| (196) | (197) | (198) |
| 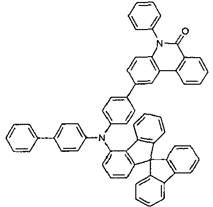 | 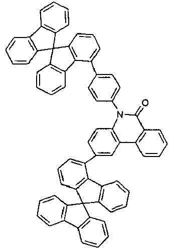 | 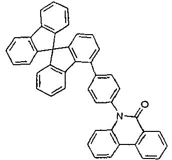 |
| (199) | (200) | (201) |

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Weiterhin lässt sich weiße Emission bevorzugt erzeugen durch Verwendung einer blauen Emissionsschicht und einer Emissionsschicht, die rot und grün emittiert, wobei diese beiden Emissionsschichten durch eine Ladungserzeugungsschicht voneinander separiert sein können.

Die Verbindung gemäß Formel (1) kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. die oben ausgeführten bevorzugten Ausführungsformen als Matrixmaterial für fluoreszierende oder phosphoreszierende Emitter, insbesondere für phosphoreszierende Emitter, und/oder in einer Lochblockierschicht und/oder in einer Elektronentransportschicht und/oder in einer elektronenblockierenden bzw. exzitonenblockierenden Schicht und/oder in einer Lochtransportschicht, je nach genauer Substitution.

In einer weiteren Ausführungsform der Erfindung enthält die organische Elektrolumineszenzvorrichtung die Verbindung gemäß Formel (1) bzw. die oben ausgeführten bevorzugten Ausführungsformen in einer optischen Auskopplungsschicht. Unter einer optischen Auskopplungsschicht wird dabei eine Schicht verstanden, die nicht zwischen der Anode und der Kathode liegt, sondern die außerhalb der eigentlichen Vorrichtung auf eine Elektrode aufgebracht wird, beispielsweise zwischen einer Elektrode und einem Substrat, um die optische Auskopplung zu verbessern.

In einer bevorzugten Ausführungsform der Erfindung wird die Verbindung gemäß Formel (1) bzw. die oben ausgeführten bevorzugten Ausführungsformen als Matrixmaterial für eine fluoreszierende oder phosphoreszierende Verbindung, insbesondere für eine phosphoreszierende Verbindung, in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine Verbindung gemäß Formel (1) als Matrixmaterial enthält.

Wenn die Verbindung gemäß Formel (1) bzw. die oben ausgeführten bevorzugten Ausführungsformen als Matrixmaterial für eine emittierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit Spinmultiplizität > 1, insbesondere aus einem angeregten Triplettzustand verstanden. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der Verbindung gemäß Formel (1) bzw. der oben ausgeführten bevorzugten Ausführungsformen und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der Verbindung gemäß Formel (1) bzw. der oben ausgeführten bevorzugten Ausführungsformen bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial. Je nach Wahl des Matrixmaterials kann auch eine geringere Emitterkonzentration bevorzugt sein, wie z. B. in der nicht offen gelegten Anmeldung EP 11002816.4 beschrieben.

Eine weitere bevorzugte Ausführungsform der vorliegenden Erfindung ist der Einsatz der Verbindung gemäß Formel (1) bzw. der oben ausgeführten bevorzugten Ausführungsformen als Matrixmaterial für einen phosphoreszierenden Emitter in Kombination mit einem weiteren Matrixmaterial. Eine weitere bevorzugte Ausführungsform der vorliegenden Erfindung ist der Einsatz der Verbindung gemäß Formel (1) bzw. gemäß den bevorzugten Ausführungsformen als Matrixmaterial für einen phosphoreszierenden Emitter in Kombination mit einem weiteren Matrixmaterial. Besonders geeignete Matrixmaterialien, welche in Kombination mit den Verbindungen gemäß Formel (1) bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Bis-carbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109, WO 2011/000455 oder der nicht offen gelegten Anmeldung EP 11007693.2, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte CarbazolDerivate, z. B. gemäß US 2009/0136779, WO 2010/050778, WO 2011/042107, WO 2011/088877 oder gemäß der nicht offen gelegten Anmeldung EP 11003232.3, Triphenylenderivaten, z. B. gemäß der nicht offen gelegten Anmeldung DE 102010048608.6, oder Lactame, z. B. gemäß den nicht offen gelegten Anmeldungen DE 102010012738.8 oder DE 102010019306.2. Ebenso kann ein weiterer phosphoreszierender Emitter, welcher kürzerwellig als der eigentliche Emitter emittiert, als Co-Host in der Mischung vorhanden sein.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Beispiele der oben beschriebenen Emitter können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In einer weiteren bevorzugten Ausführungsform der Erfindung wird die Verbindung gemäß Formel (1) bzw. die oben ausgeführten bevorzugten Ausführungsformen als Elektronentransportmaterial in einer Elektronentransport- oder Elektroneninjektionsschicht eingesetzt. Dabei kann die emittierende Schicht fluoreszierend oder phosphoreszierend sein. Wenn die Verbindung als Elektronentransportmaterial eingesetzt wird, kann es bevorzugt sein, wenn sie dotiert ist, beispielsweise mit Alkalimetallkomplexen, wie z. B. LiQ (Lithiumhydroxychinolinat).

In nochmals einer weiteren bevorzugten Ausführungsform der Erfindung wird die Verbindung gemäß Formel (1) bzw. die oben ausgeführten bevorzugten Ausführungsformen in einer Lochblockierschicht eingesetzt. Unter einer Lochblockierschicht wird eine Schicht verstanden, die auf Kathodenseite direkt an eine emittierende Schicht angrenzt.

Es ist weiterhin möglich, die Verbindung gemäß Formel (1) bzw. die oben ausgeführten bevorzugten Ausführungsformen sowohl in einer Lochblockierschicht bzw. Elektronentransportschicht als auch als Matrix in einer emittierenden Schicht zu verwenden.

In nochmals einer weiteren Ausführungsform der Erfindung wird die Verbindung gemäß Formel (1) bzw. die oben ausgeführten bevorzugten Ausführungsformen in einer Lochtransportschicht bzw. in einer Elektronenblockierschicht bzw. Exzitonenblockierschicht eingesetzt.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden. Diese Verfahren eignen sich insbesondere auch für Oligomere, Dendrimere und Polymere.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Ein weiterer Gegenstand der vorliegenden Erfindung sind die oben als bevorzugt aufgeführten Verbindungen der folgenden Formel (1'), 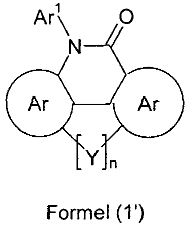 wobei für die verwendeten Symbole und Indizes gilt:
- Ar: ist bei jedem Auftreten gleich oder verschieden eine Aryl- oder Heteroarylgruppe mit 5 bis 13 aromatischen Ringatomen, welche durch einen oder mehrere Reste R substituiert sein kann;
- Ar¹: ist ein aromatisches oder heteroaromatisches Ringsystem mit 5 bis 24 aromatischen Ringatomen, welches durch einen oder mehrere Reste R substituiert sein kann und welches keine Aryl- oder Heteroarylgruppen mit mehr als zwei direkt aneinander kondensierten aromatischen Sechsringen aufweist;
- Y: ist -C(=O)-N(Ar¹)-, -C(=O)-O-, -CR¹=CR¹-, -CR¹=N-, C(R¹)₂, NR¹, O, S, C(=O), C(=S), C(=NR¹), C(=C(R¹)₂), Si(R¹)₂, BR¹, PR¹, P(=O)R¹, SO oder SO₂;
- R, R¹: ist bei jedem Auftreten gleich oder verschieden ausgewählt aus der Gruppe bestehend aus H, D, F, I, N(Ar²)₂, C(=O)Ar², C(=O)R², P(=O)(Ar²)₂, einer geradkettigen Alkylgruppe mit 1 bis 40 C-Atomen oder einer verzweigten oder cyclischen Alkylgruppe mit 3 bis 40 C-Atomen oder einer Alkenyl- oder Alkinylgruppe mit 2 bis 40 C-Atomen, die jeweils mit einem oder mehreren Resten R² substituiert sein kann, wobei eine oder mehrere nicht-benachbarte CH₂-Gruppen durch R²C=CR², C≡C, Si(R²)₂, C=O, C=S, C=NR², P(=O)(R²), SO, SO₂, S oder CONR² ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R² substituiert sein kann, einer Aryloxy- oder Heteroaryloxygruppe mit 5 bis 60 aromatischen Ringatomen, die mit einem oder mehreren Resten R² substituiert sein kann, oder einer Kombination dieser Systeme, wobei optional zwei oder mehr benachbarte Substituenten R bzw. zwei oder mehr benachbarte Substituenten R¹ ein monocyclisches oder polycyclisches, aliphatisches, aromatisches oder heteroaromatisches Ringsystem bilden können, das mit einem oder mehreren Resten R² substituiert sein kann; weiterhin kann auch ein Rest R an Ar¹ mit einem Rest R an Ar ein aliphatisches Ringsystem bilden; mit der Maßgabe, dass durch Ringbildung der Reste R bzw. R¹ keine Aryl- bzw. Heteroarylgruppe mit mehr als zwei direkt aneinander kondensierten Arylgruppen entsteht;
- Ar²: ist bei jedem Auftreten gleich oder verschieden ein aromatisches oder heteroaromatisches Ringsystem mit 5-30 aromatischen Ringatomen, das mit einem oder mehreren nicht-aromatischen Resten R² substituiert sein kann; dabei können zwei Reste Ar², welche an dasselbe N-Atom oder P-Atom binden, auch durch eine Einfachbindung oder eine Brücke, ausgewählt aus N(R²), C(R²)₂ oder O, miteinander verbrückt sein;
- R²: ist ausgewählt aus der Gruppe bestehend aus H, D, F, einem aliphatischem Kohlenwasserstoffrest mit 1 bis 20 C-Atomen, einem aromatischem oder heteroaromatischem Ringsystem mit 5 bis 30 aromatischen Ringatomen, in dem ein oder mehrere H-Atome durch D, F, Cl, Br, I oder CN ersetzt sein können, wobei zwei oder mehr benachbarte Substituenten R³ miteinander ein mono- oder polycyclisches, aliphatisches, aromatisches oder heteroaromatisches Ringsystem bilden können;
- n: ist bei jedem Auftreten gleich oder verschieden 0 oder 1, wobei n = 1 bedeutet, dass keine Gruppe Y vorhanden ist und statt dessen in den Positionen an Ar, in denen in Formel (1) Y gebunden ist, ein Substituent R gebunden ist oder ein Heteroatom der Gruppe Ar vorhanden ist;
mit der Maßgabe, dass die Reste an Ar oder Ar¹ keine Lactamgruppe enthalten;
dabei sind die folgenden Verbindungen von der Erfindung ausgenommen:

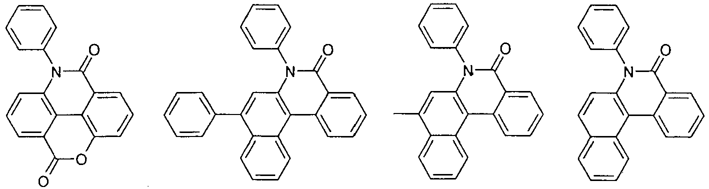

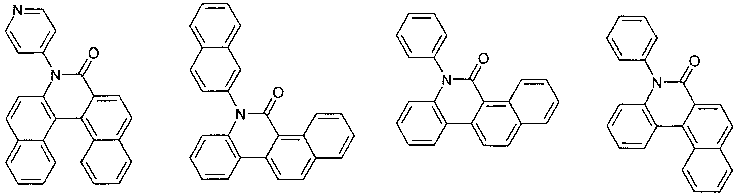

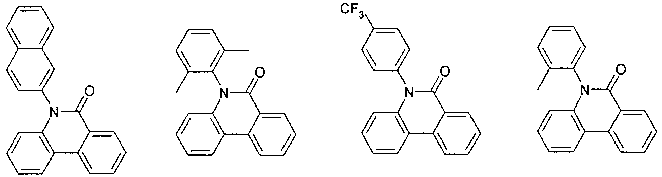

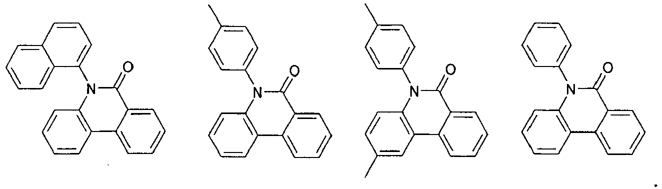

Für die erfindungsgemäßen Verbindungen der Formel (1') gelten dieselben Bevorzugungen, wie oben für die Verbindungen der Formel (1) angegeben.

Die Verbindungen der Formel (1) bzw. (1') bzw. die bevorzugten Ausführungsformen können nach dem Fachmann bekannten Syntheseschritten dargestellt werden, wie in Schema 1 bis 3 schematisch dargestellt.

Aus der Literatur ist ein Verfahren zur Synthese von Aryl-substituierten Lactamen aus dem am Stickstoff unsubstituierten Lactam mit einem Aryliodid (z. B. gemäß WO 2007/062230; Schema 1a) durch Ullmann-Kupplung bekannt. Weitere literaturbekannte Synthesen gehen vom entsprechenden Amid aus, welches zum Lactam cyclisiert wird (z. B. Chemistry - An Asian Journal 2010, 5(9), 2113-2123; Organic Reactions (Hoboken, NJ, United States), 48, 1996; Schema 1b und 1c).

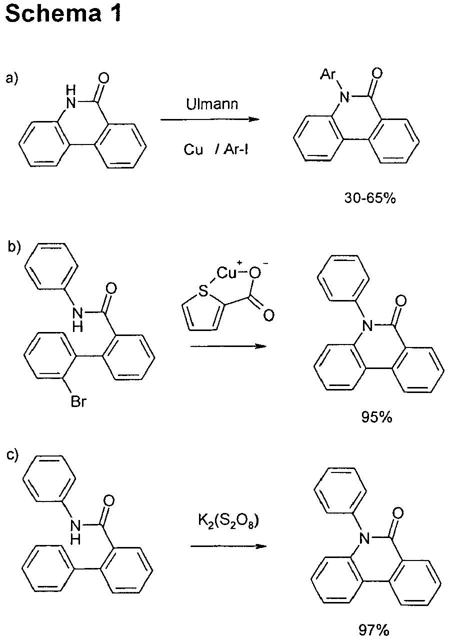

Im Folgenden wird die Synthese der Verbindungen durch eine neue Syntheseroute, die hohe Ausbeuten liefert, beschrieben, wie in Schema 2 und 3 dargestellt. Bei diesen Verfahren wird nicht der Stickstoff eines unsubstituierten Lactams substituiert oder ein Amid zu einem Lactam funktionalisiert, sondern es findet eine Ringschlussreaktion zwischen zwei Arylgruppen statt, wobei eine der Arylgruppen an die Carbonylgruppe eines Amids und die andere Arylgruppe an den Stickstoff eines Amids gebunden ist. Dabei kann die Cyclisierung entweder Palladium-katalysiert (Schema 2) oder radikalisch unter Katalyse eines Zinnhydrids durchgeführt werden (Schema 3). Weiterhin können beide Synthesemethoden sowohl am Amin durchgeführt werden, welches dann in einem Folgeschritt zum Lactam oxidiert wird, wie in Schema 2 dargestellt, oder sie können am Amid durchgeführt werden, wie in Schema 3 dargestellt. Für beide Reaktionstypen werden bei der Ringschlussreaktion sehr gute Ausbeuten erhalten.

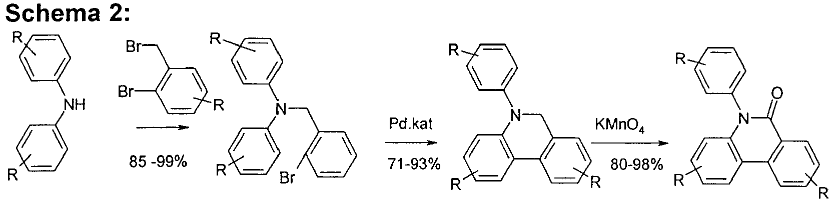

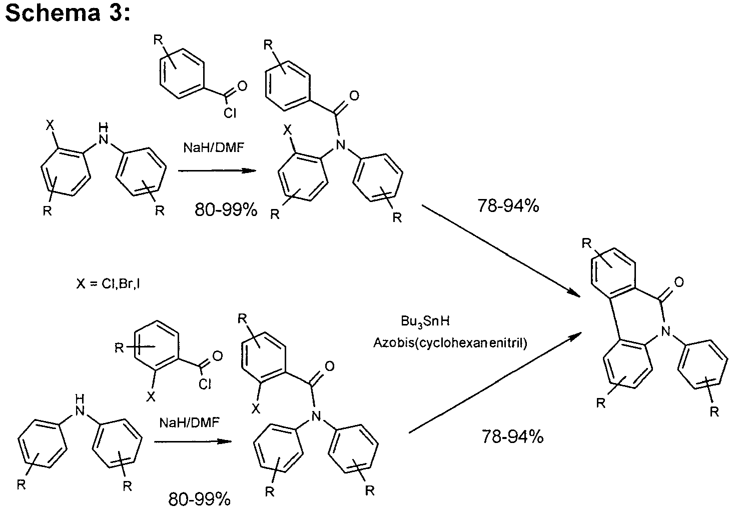

Eine Methode zur Funktionalisierung der erfindungsgemäßen Verbindungen durch Halogenieren, beispielsweise Bromierung mit NBS, gefolgt von einer metallkatalysierten Kupplungsreaktion, beispielsweise eine Suzuki-Kupplung oder einer Hartwig-Buchwald-Kupplung, ist in Schema 4 dargestellt.

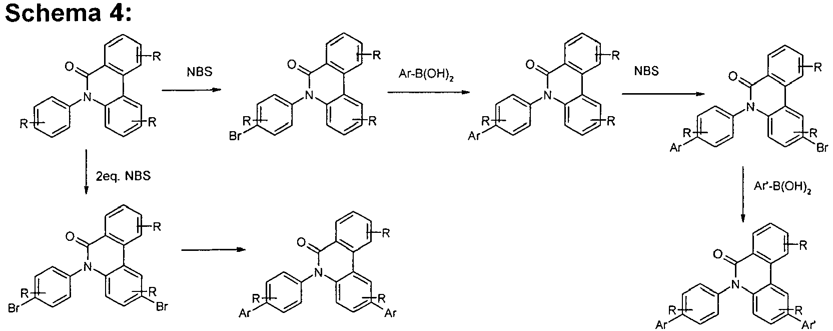

Ein weiterer Gegenstand der vorliegenden Erfindung ist ein Verfahren zur Herstellung einer Verbindung gemäß Formel (1'), umfassend:
a) die Bindungsknüpfung zwischen dem Stickstoff eines Lactams und Ar¹; oder
b) die Bindungsknüpfung zwischen dem Stickstoff eines Amids und Ar; oder
c) die Bindungsknüpfung zwischen den beiden Gruppen Ar, die über eine Amidgruppe miteinander verknüpft sind; oder
d) die Bindungsknüpfung zwischen den beiden Gruppen Ar, die über eine Methylenamingruppe miteinander verknüpft sind, gefolgt von einer Oxidation zum entsprechenden Lactam.

Ein weiterer Gegenstand der vorliegenden Erfindung ist die Verwendung der oben ausgeführten erfindungsgemäßen Verbindungen gemäß Formel (1') in einer elektronischen Vorrichtung, insbesondere in einer organischen Elektrolumineszenzvorrichtung.

Die erfindungsgemäßen Verbindungen und die erfindungsgemäßen organischen Elektrolumineszenzvorrichtungen zeichnen sich durch einen oder mehrere der folgenden überraschenden Vorteile gegenüber dem Stand der Technik aus:
1. Die erfindungsgemäßen Verbindungen bzw. Verbindungen gemäß Formel (1) bzw. die oben ausgeführten bevorzugten Ausführungsformen, eingesetzt als Matrixmaterial für fluoreszierende oder phosphoreszierende Emitter, führen zu sehr hohen Effizienzen sowie zu langen Lebensdauern. Dies gilt insbesondere, wenn die Verbindungen als Matrixmaterial für einen rot oder grün phosphoreszierenden Emitter eingesetzt werden.
2. Die erfindungsgemäßen Verbindungen weisen eine hohe thermische Stabilität auf.
3. Die erfindungsgemäßen Verbindungen, eingesetzt in organischen Elektrolumineszenzvorrichtungen, führen zu hohen Effizienzen und zu steilen Strom-Spannungs-Kurven mit niedrigen Einsatzspannungen.
4. Auch bei Verwendung als Elektronentransportmaterial führen die erfindungsgemäßen Verbindungen zu sehr guten Eigenschaften in Bezug auf die Effizienz, die Lebensdauer und die Betriebsspannung von organischen Elektrolumineszenzvorrichtungen.
5. Die Verbindungen der Formel (1) lassen sich einfach und in hoher Ausbeute synthetisieren.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre durchgeführt. Die Edukte können von den Firmen ALDRICH bzw. ABCR (Palladium(II)acetat, Tri-o-tolylphosphin, Anorganika, Lösemittel) bezogen werden. Die Angaben bei den literaturbekannten Edukten sind die CAS-Nummern.

### Beispiel 1: 2-Brom-N,N-bis(biphenyl)-benzolmethanamin

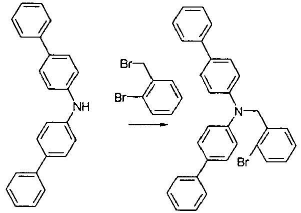

9.7 g (0.243 mol) NaH 60%ig in Mineralöl werden in 500 mL Dimethylformamid unter Schutzatmosphäre gelöst. 60 g (0.106 mol) N,N-Bis(4-biphenyl)amin werden in 500 mL DMF gelöst und zu der Reaktionsmischung zugetropft. Nach 1 h bei Raumtemperatur wird eine Lösung von 60.6 (242 mmol)2-Brombenzylbromid in 500 mL DMF zugetropft. Das Reaktionsgemisch wird 1 h bei Raumtemperatur gerührt. Nach dieser Zeit wird das Reaktionsgemisch auf Eis gegossen und dreimal mit Dichlormethan extrahiert. Die vereinigten organischen Phasen werden über Na₂SO₄ getrocknet und eingeengt. Der Rückstand wird mit Toluol heiß extrahiert und aus Toluol / n-Heptane umkristallisiert. Die Ausbeute beträgt 80 g (93 %).

Analog werden folgende Verbindungen erhalten:

| **Bsp**. | **Edukt 1** | **Edukt 2** | **Produkt** | **Ausbeute** |
|---|---|---|---|---|
| 1a | 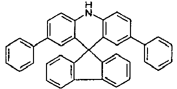 | 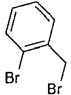 | 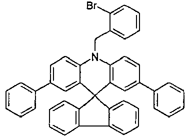 | 88 % |
| | 1303969-38-1 | 3433-80-5 | | |
| 1b | 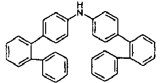 | 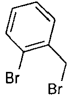 | 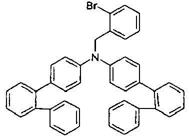 | 87 % |
| | 1222634-01-6 | 3433-80-5 | | |
| 1c | 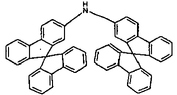 | 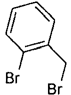 | 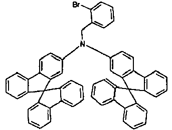 | 91 % |
| | 932731-04-9 | 3433-80-5 | | |
| 1d | 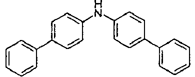 | 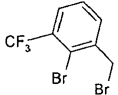 | 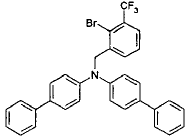 | 90 % |
| | | 1214372-35-6 | | |
| 1e | 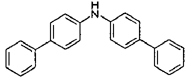 | 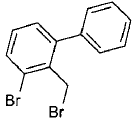 | 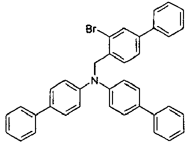 | 93% |
| | | 172976-02-2 | | |
| 1f | 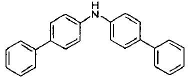 | 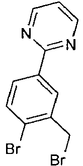 | 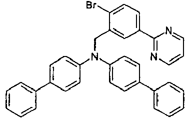 | 82 % |
| | | 202805-71-8 | | |
| 1g | 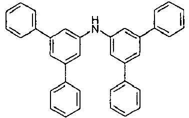 | 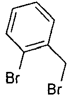 | 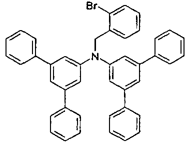 | 87% |
| | 1290039-78-9 | 3433-80-5 | | |
| 1h | 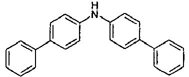 | 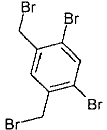 | 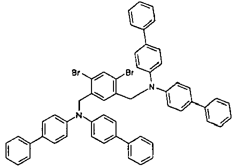 | 79% |
| | | 35510-03-3 | | |
| 1i | 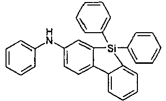 | 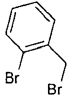 | 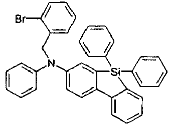 | 79% |
| | 860465-15-2 | 3433-80-5 | | |
| 1j | 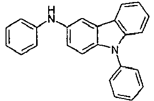 | 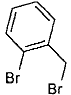 | 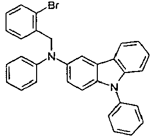 | 89% |
| | 894791-43-6 | 3433-80-5 | | |
| 1k | 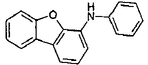 | 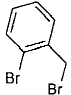 | 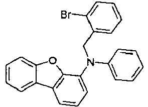 | 84% |
| | 743453-07-8 | 3433-80-5 | | |
| 1l | 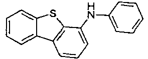 | 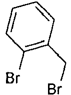 | 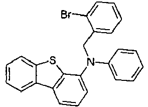 | 86% |
| | 1300028-91-4 | 3433-80-5 | | |

### Beispiel 2: 5-Biphenyl-4-yl-2-phenyl-5,6-dihydro-phenanthridin

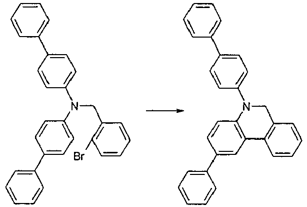

47 g (0.158 mol) 2-Brom-N,N-bisbiphenyl-benzolmethanamin werden in 500 mL Dimethylformamid unter Schutzatmosphäre gelöst. Zur dieser Lösung werden17.3 g (0.075 mol) Benzyltrimethylammoniumbromid und 31.28 g (0.226 mol) Kaliumcarbonat werden zugegeben. Anschließend wird unter Schutzgas 5.08 g (0.022 mol) Pd(OAc)₂ zugegeben, und das Gemisch wird bei 120 °C für 9 h gerührt. Nach dieser Zeit wird das Reaktionsgemisch auf Raumtemperatur gekühlt und mit Dichlormethan extrahiert. Die vereinigten organischen Phasen werden über Na₂SO₄ getrocknet und eingeengt. Der Rückstand wird aus n-Heptan umkristallisiert. Die Ausbeute beträgt 51 g (84 %).

Analog werden folgende Verbindungen erhalten:

| **Bsp**. | **Edukt 1** | **Produkt** | **Ausbeute** |
|---|---|---|---|
| 2a | 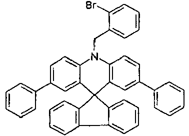 | 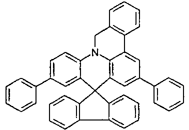 | 83 % |
| 2b | 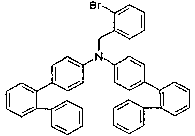 | 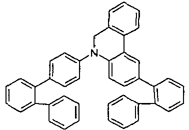 | 82 % |
| 2c | 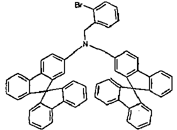 | 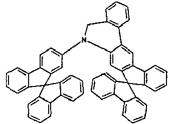 | 80 % |
| 2d | 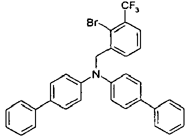 | 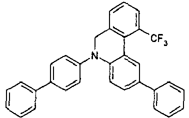 | 78 % |
| 2e | 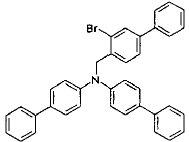 | 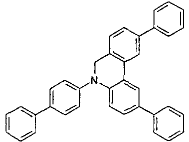 | 90% |
| 2f | 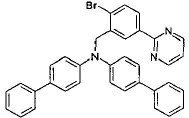 | 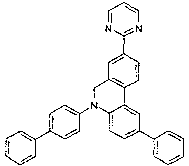 | 69 % |
| 2g | 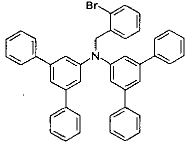 | 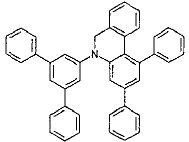 | 67% |
| 2h | 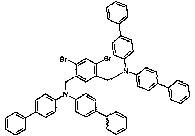 | 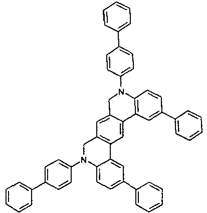 | 71% |
| 2i | 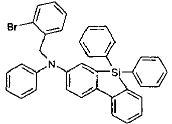 | 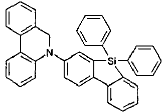 | 77% |
| 2j | 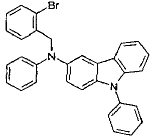 | 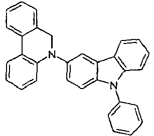 | 60% |
| 2k | 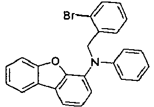 | 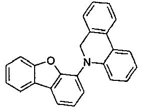 | 53% |
| 2l | 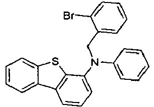 | 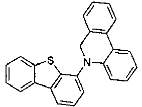 | 55% |

### Beispiel 3: 5-Biphenyl-4-yl-2-phenyl-6(5H)-phenanthridinon

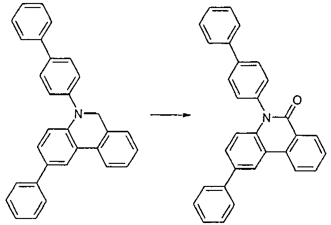

16 g (0.039 mol) 5-Biphenyl-4-yl-2-phenyl-5,6-dihydrophenanthridin werden in 300 mL Dichlormethan gelöst. Zur diese Lösung werden und 62.13 g (0.393 mol) Kaliumpermanganat portionsweise zugegeben und zwei Tage bei Raumtemperatur gerührt. Nach dieser Zeit wird das restliche Kaliumpermanganat abfiltriert, die Lösung eingeengt und chromatographisch gereinigt (Laufmittel: Heptan/Dichloromethan, 5:1). Der Rückstand wird aus n-Heptan umkristallisiert. Die Ausbeute beträgt 14 g (88 %).

Analog werden folgende Verbindungen erhalten:

| **Bsp.** | **Edukt 1** | **Produkt** | **Ausbeute** |
|---|---|---|---|
| 3a | 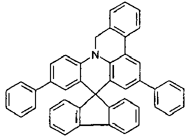 | 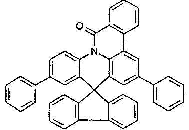 | 83 % |
| 3b | 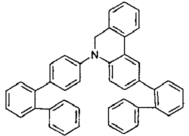 | 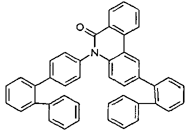 | 82 % |
| 3c | 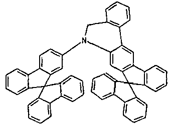 | 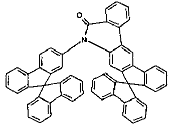 | 80% |
| 3d | 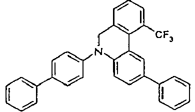 | 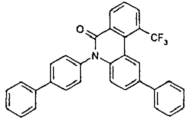 | 78 % |
| 3e | 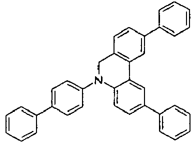 | 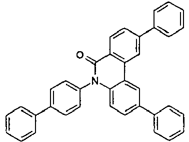 | 90% |
| 3f | 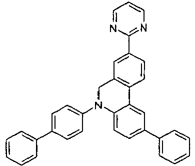 | 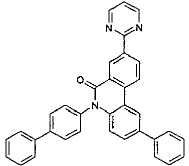 | 69% |
| 3g | 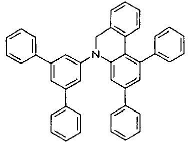 | 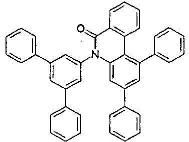 | 67% |
| 3h | 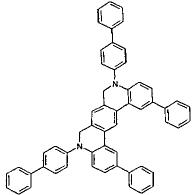 | 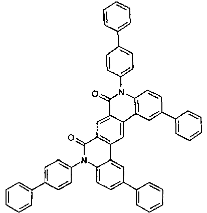 | 71% |
| 3i | 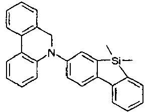 | 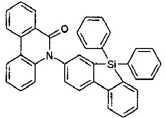 | 77% |
| 3j | 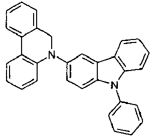 | 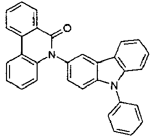 | 72% |
| 3k | 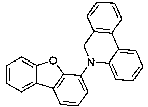 | 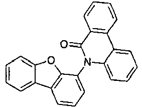 | 78% |
| 3l | 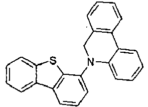 | 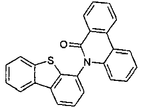 | 76% |

### Beispiel 4: 2-Brom-3-nitrobenzoesäuremethylester

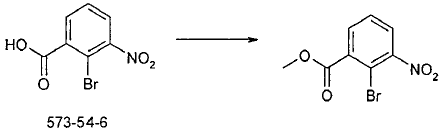

100 g (406 mmol) 2-Brom-3-nitrobenzoesäure, 22 g (609 mmol) HCl konz. und 1 L Methanol werden 24 h unter Rückfluss erhitzt. Nach Erkalten wird der ausgefallene Feststoff abgesaugt, mit Wasser und Ethanol gewaschen und getrocknet. Der Rückstand wird aus Ethanol umkristallisiert. Ausbeute: 94,6g (363 mmol), 89,5 % der Theorie.

### Beispiel 5: 1,1'-Biphenyl-6,6'-dinitro-2,2'-dimethylcarbonsäureester

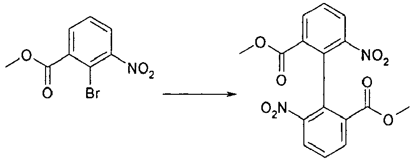

84 g (323 mmol) 2-Brom-3-nitrobenzoesäuremethylester und 61.5 g (969 mmol) Kupferpulver werden mit 1 L DMF versetzt und das Gemisch bei 120 °C für 1 h gerührt. Der Ansatz wird abgekühlt und das Kupfer abfiltriert. Die Lösung wird mit Wasser verdünnt, mit Essigester zweimal extrahiert, die vereinigten organischen Phasen über Na₂SO₄ getrocknet und einrotiert. Der Rückstand wird aus Methanol umkristallisiert. Die Ausbeute beträgt 47.4 g (131.5 mmol) 81.5 % der Theorie.

### Beispiel 6: 1,1'-Biphenyl- 6.6'-dinitro-2,2'-carbonsäure

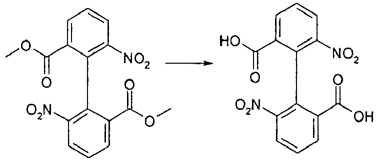

42 g (116.5 mmol) 1,1'-Biphenyl-6.6'-dinitro-2,2'-dimethylcarbonsäure-ester und 21 g (524 mmol) NaOH werden mit 100 ml Wasser und 100 ml Ethanol versetzt und das Gemisch bei 90 °C für 2 h gerührt. Der Ansatz wird abgekühlt, mit 5M HCl sauer gestellt und 30 min. gerührt. Der ausgefallene Feststoff abgesaugt. Der Rückstand wird aus Heptan umkristallisiert. Ausbeute: 33.6 g (332.2 mmol), 86 % der Theorie.

### Beispiel 7: Pyrido[2,3,4,5-Imn]phenanthridin-5,10-dion

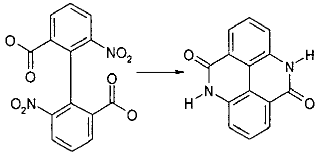

40 g (129 mmol) 11,1'-Biphenyl-6,6'-dinitro-2,2'-carbonsäure und 50 g (931 mmol) Eisenpulver werden mit 500 ml Essigsäure bei 80 °C für 12 h gerührt. Der Ansatz wird abgekühlt und der ausgefallene Feststoff abgesaugt. Der Rückstand wird aus DMF umkristallisiert. Ausbeute: 19 g (84.3 mmol), 70 % der Theorie.

### Beispiel 8: 4,9 Dihenyl-4,9-dihydro-Pyrido[2,3,4,5-Imn]phenanthridin-5,10-dion

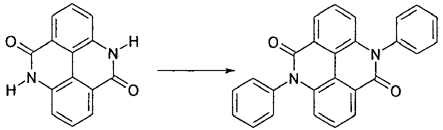

23 g (100 mmol) Pyrido[2,3,4,5-Imn]phenanthridin-5,10-dion und 61.2 g (300 mmol) 4-lodbenzol und 2.3 g (20 mmol) L-Prolin werden in 100 ml bei 150 °C für 30 h gerührt Die Lösung wird mit Wasser verdünnt und mit Essigester zweimal extrahiert, die vereinigten organischen Phasen über Na₂SO₄ getrocknet und einrotiert. Der Rückstand wird chromatographisch (EtOAc/Hexan: 2/3) gereinigt. Die Ausbeute beträgt 20 g (52 mmol), 55 % der Theorie.

Analog werden folgende Verbindungen erhalten:

| **Bsp**. | **Edukt 1** | **Edukt 1** | **Produkt** | **Ausbeute** |
|---|---|---|---|---|
| 8a | 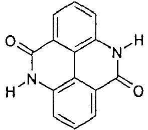 | 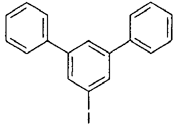 | 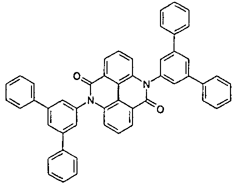 | 57% |
| 8b | 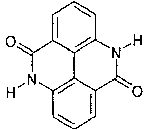 | 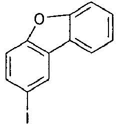 | 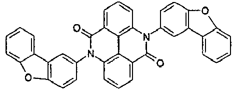 | 56% |

### Beispiel 9: 6-[1,1';3',1"]-Terphenyl-5'-yl-6H-benzo[c][2,6]naphthyridin-5-on

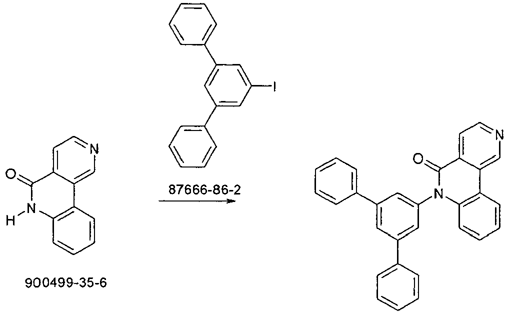

19,6 g (100 mmol) Benzo[c]-2,6-napthyridinon, 106 g (300 mmol) 5'-lod-[1,1';3',1"]terphenyl und 2.3 g (20 mmol) L-Prolin werden in 100 ml bei 150 °C für 30 h gerührt Die Lösung wird mit Wasser verdünnt, mit Essigester zweimal extrahiert, die vereinigten organischen Phasen über Na₂SO₄ getrocknet und einrotiert. Der Rückstand wird chromatographisch (EtOAc / Hexan: 2/3) gereinigt. Die Ausbeute beträgt 25 g (59 mmol) 60 % der Theorie.

Analog werden folgende Verbindungen erhalten:

| **Bsp**. | **Edukt 1** | **Edukt 1** | **Produkt** | **Ausbeute** |
|---|---|---|---|---|
| 9a | 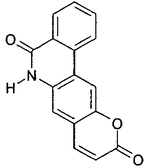 | 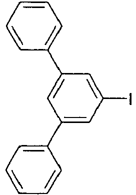 | 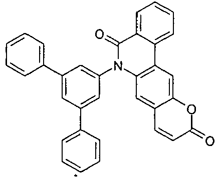 | 56% |
| | 17606-34-6 | 87666-86-2 | | |
| 9b | 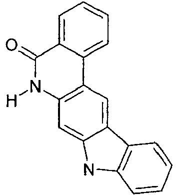 | 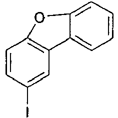 | 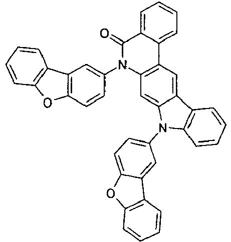 | 54 |
| | 164261-68-1 | | | |
| 9c | 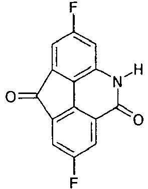 | 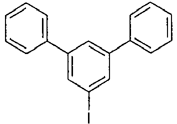 | 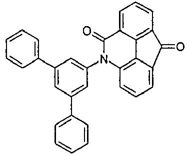 | 58% |
| | 391248-47-8 | | | |
| 9d | 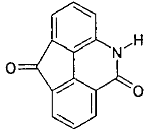 | 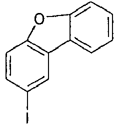 | 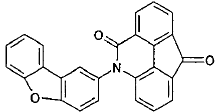 | 53% |
| | 61479-80-9 | | | |
| 9e | 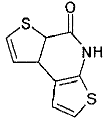 | 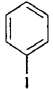 | 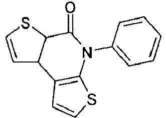 | 59% |
| | 40197-38-4 | | | |
| 9f | 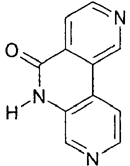 | 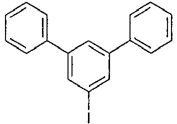 | 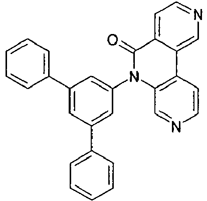 | 62% |
| | 1186039-92-8 | | | |
| 9g | 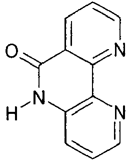 | 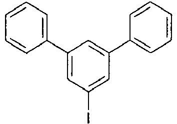 | 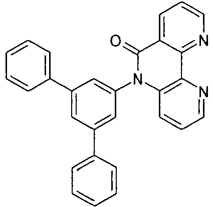 | 63% |
| | 851066-63-2 | | | |

### Beispiel 10: 2-(2-Brom-benzyl)-diphenyl-amin

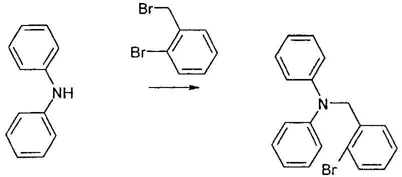

12 g (0.307 mol) NaH (60%ig in Mineralöl) werden in 600 mL Dimethylformamid unter Schutzatmosphäre gelöst. 40 g (0.263 mol) N,N-Diphenylamin werden in 600 mL DMF gelöst und zu der Reaktionsmischung zugetropft. Nach 1 h bei Raumtemperatur wird eine Lösung von 98.7 (395 mmol) 2-Brombenzylbromid in 500 mL DMF zugetropft. Das Reaktionsgemisch wird 1 h bei Raumtemperatur gerührt, dann auf Eis gegossen und dreimal mit Dichlormethan extrahiert. Die vereinigten organischen Phasen werden über Na₂SO₄ getrocknet und eingeengt. Der Rückstand wird mit Toluol heiß extrahiert und aus Toluol / n-Heptane umkristallisiert. Die Ausbeute beträgt 71 g (89 %).

Analog werden die folgenden Verbindungen erhalten:

| **Bsp.** | **Edukt 1** | **Edukt 2** | **Produkt** | **Ausbeute** |
|---|---|---|---|---|
| 10a | 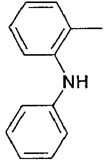 | 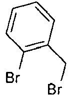 | 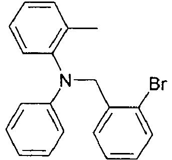 | 85 % |
| | 1205-39-6 | 3433-80-5 | | |
| 10b | 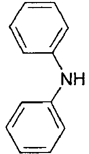 | 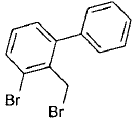 | 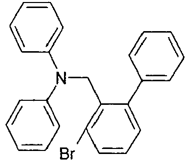 | 91 % |
| | | 172976-02-2 | | |

### Beispiel 11: 5-Phenyl-5,6-dihydrophenanthridin

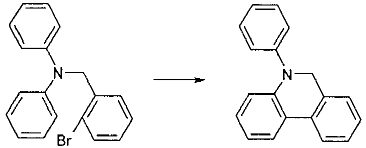

10 g (0.295 mol) (2-Brom-benzyl)-diphenyl-amin werden in 500 mL Dimethylformamide unter Schutzatmosphäre gelöst. Zur diese Lösung werden 3.4 g (0.075 mol) Benzyltrimethylammoniumbromid und 6.1 g (0.443 mol) Kaliumcarbonat zugegeben. Anschließend wird unter Schutzgas 0.99 g (0.004 mol) Pd(OAC)₂ zugegeben und das Gemisch wird bei 120 °C für 9 h gerührt. Nach dieser Zeit wird das Reaktionsgemisch auf Raumtemperatur gekühlt und mit Dichlormethan extrahiert. Die vereinigten organischen Phasen werden über Na₂SO₄ getrocknet und eingeengt. Der Rückstand wird aus n-Heptan umkristallisiert. Die Ausbeute beträgt 6 g (80 %).

Analog werden die folgenden Verbindungen erhalten:

| **Bsp.** | **Edukt 1** | **Produkt** | **Ausbeute** |
|---|---|---|---|
| 11a | 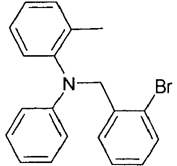 | 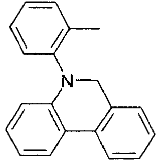 | 79 % |
| 11b | 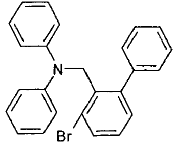 | 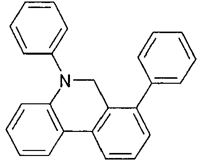 | 80 % |

### Beispiel 12: 5-Phenyl-5H-phenanthridin-6-on

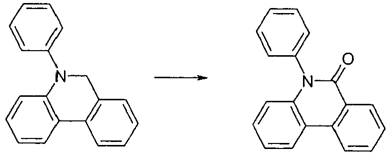

10 g (0.039 mol) 5-Phenyl-5,6-dihydrophenanthridin werden in 300 mL Dichlormethan gelöst. Zur diese Lösung werden 62.13 g (0.393 mol) Kaliumpermangenat portionsweise werden zugegeben und zwei Tage bei Raumtemperatur gerührt. Nach dieser Zeit wird das restliche Kaliumpermanganat abfiltiert, die Lösung eingeengt und chromatographisch gereinigt (Laufmittel: Heptan/Dichloromethan, 5:1) .Der Rückstand wird aus n-Heptan umkristallisiert. Die Ausbeute beträgt 8.2 g (80 %).

Analog werden die folgenden Verbindungen erhalten:

| **Bsp.** | **Edukt 1** | **Produkt** | **Ausbeute** |
|---|---|---|---|
| 12a | 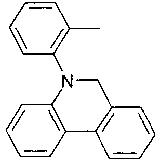 | 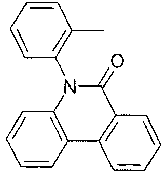 | 83 % |
| 12b | 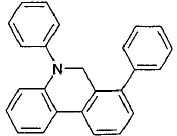 | 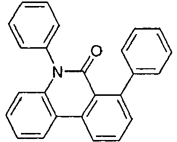 | 82 % |

### Beispiel 13: 5-(4-Brom-phenyl)-5H-phenanthridin-6-on

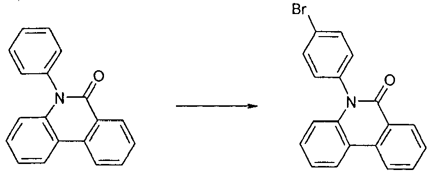

6.0 g (22.2 mmol) 5-Phenyl-5H-phenanthridin-6-on werden in 150 mL CH₂Cl₂ vorgelegt. Anschließend tropft man unter Lichtausschluss bei -15 °C eine Lösung aus 4 g (22.5 mmol) NBS in 100 ml Acetonitril zu, lässt auf Raumtemperatur kommen und rührt 4 h weiter bei dieser Temperatur. Anschließend wird die Mischung mit 150 mL Wasser versetzt und mit CH₂Cl₂ extrahiert. Die organische Phase wird über MgSO₄ getrocknet und die Lösungsmittel im Vakuum entfernt. Das Produkt wird mit Hexan heiß ausgerührt und abgesaugt. Ausbeute: 7.4 g (21 mmol), 80 % d. Th., Reinheit nach ¹H-NMR ca. 96 %.

Analog werden die folgenden Verbindungen erhalten:

| **Bsp.** | **Edukt 1** | **Produkt 1** | **Ausbeute** |
|---|---|---|---|
| 13a | 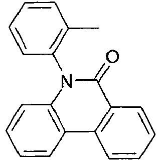 | 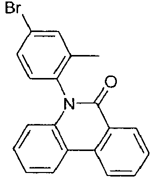 | 65 % |
| 13b | 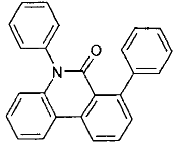 | 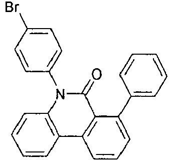 | 83% |

Analog werden die folgenden Verbindungen mit 2 Äquivalenten NBS in Chloroform als Lösungsmittel erhalten:

| **Bsp.** | **Edukt 1** | **Produkt 1** | **Ausbeute** |
|---|---|---|---|
| 13c | 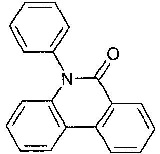 | 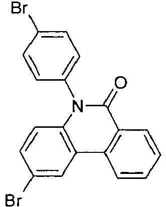 | 87 |
| 13d | 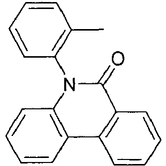 | 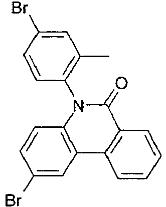 | 82 % |
| 13f | 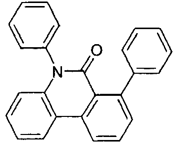 | 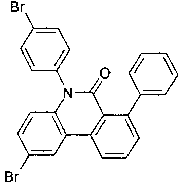 | 83% |

### Beispiel 14: 5-(4-Dibenzofuran-4-yl-phenyl)-5H-phenanthridin-6-on

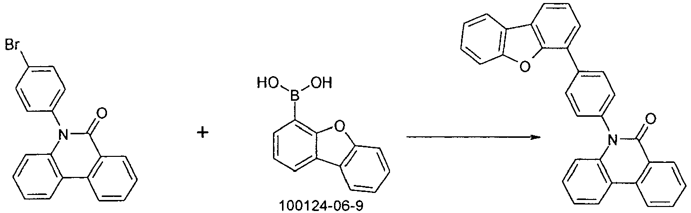

23 g (110.0 mmol) 4-Dibenzofuranboronsäure, 38 g (110.0 mmol) 5-(4-Brom-phenyl)-5H-phenanthridin-6-on und 44.6 g (210.0 mmol) Trikaliumphosphat werden in 500 mL Toulol, 500 mL Dioxan und 500 mL Wasser suspendiert. Zu dieser Suspension werden 913 mg (3.0 mmol) Tri-o-tolylphosphin und dann 112 mg (0.5 mmol) Palladium(II)acetat gegeben, und die Reaktionsmischung wird 16 h unter Rückfluss erhitzt. Nach Erkalten wird die organische Phase abgetrennt, über Kieselgel filtriert, dreimal mit je 200 mL Wasser gewaschen und anschließend zur Trockene eingeengt. Der Rückstand wird aus Toluol und aus Dichlormethan / *iso*-Propanol umkristallisiert und abschließend im Hochvakuum sublimiert, Reinheit beträgt 99.9 %. Die Ausbeute beträgt 38 g (92 mmol), entsprechend 84 % der Theorie.

Analog werden die folgenden Verbindungen erhalten:

| **Bsp.** | **Edukt 1** | **Edukt 2** | **Produkt** | **Ausbeute** |
|---|---|---|---|---|
| 14a | 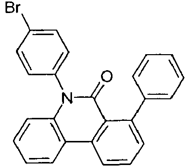 | 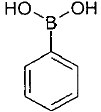 | 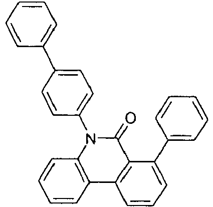 | 90 % |
| 14b | 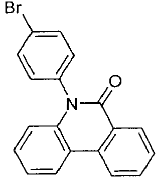 | 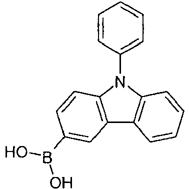 | 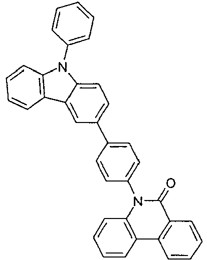 | 81% |
| | | 854952-58-2 | | |
| 14c | 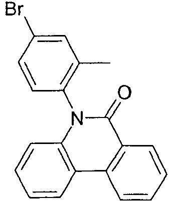 | 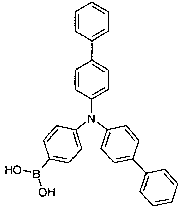 | 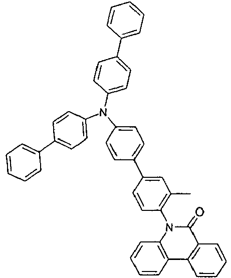 | 84% |
| | | 943836-24-6 | | |
| 14d | 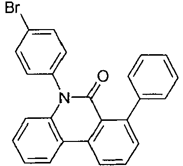 | 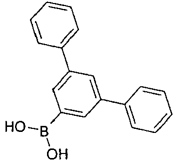 | 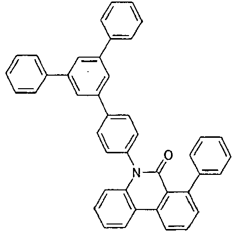 | 88% |
| | | 128388-54-5 | | |
| 14e | 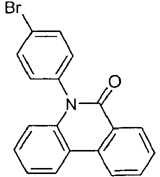 | 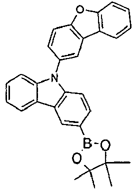 | 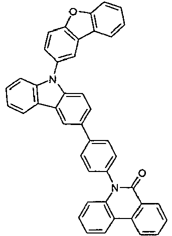 | 67% |
| | | 1338488-91-7 | | |
| 14f | 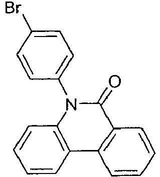 | 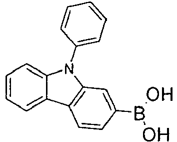 | 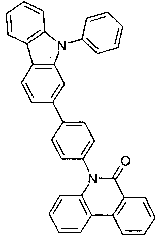 | 75% |
| | | 1001911-63-2 | | |
| 14g | 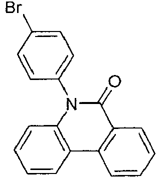 | 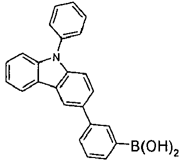 | 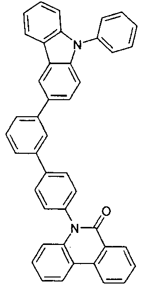 | 76% |
| | | 854952-60-6 | | |
| 14h | 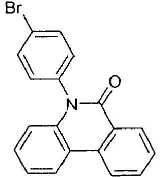 | 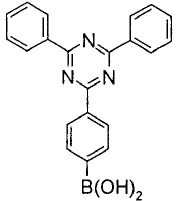 | 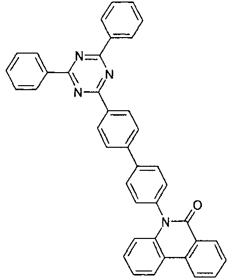 | 83% |
| | | 1313018-07-3 | | |

Analog werden die folgenden Verbindungen mit 0,5 Äquivalenten des Lactams erhalten:

| **Bsp.** | **Edukt 1** | **Edukt 2** | **Produkt** | **Ausbeute** |
|---|---|---|---|---|
| 14i | 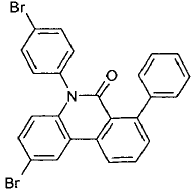 | 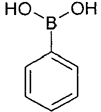 | 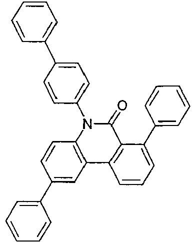 | 93 % |
| 14j | 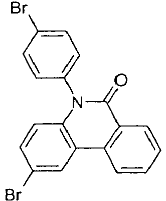 | 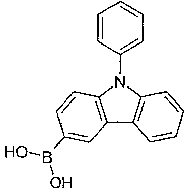 | 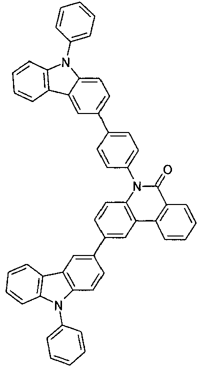 | 84% |
| | | 854952-58-2 | | |
| 14k | 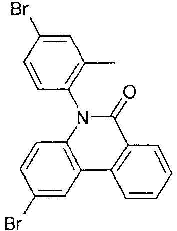 | 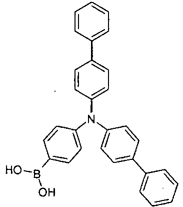 | 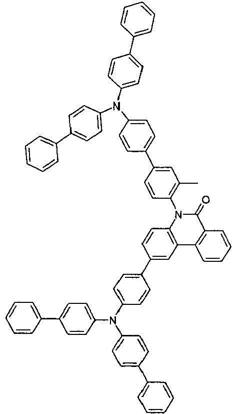 | 84% |
| | | 943836-24-6 | | |
| 14l | 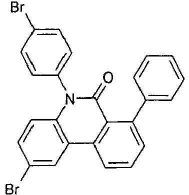 | 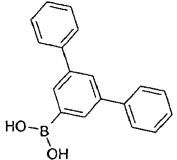 | 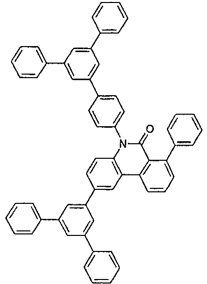 | 88% |
| | | 128388-54-5 | | |
| 14m | 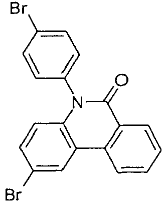 | 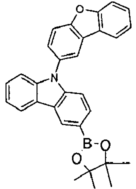 | 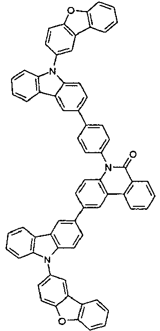 | 75% |
| | | 1338488-91-7 | | |
| 14n | 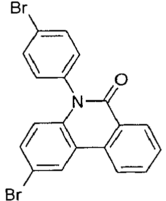 | 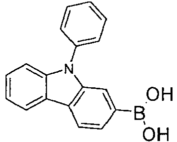 | 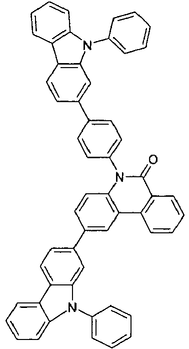 | 72% |
| | | 1001911-63-2 | | |
| 14o | 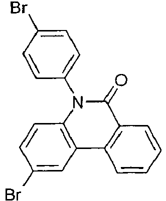 | 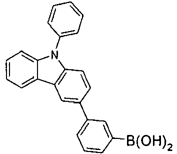 | 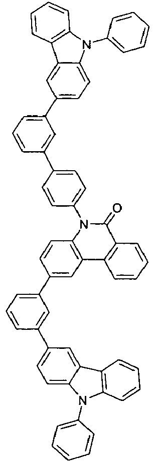 | 70% |
| | | 854952-60-6 | | |
| 14p | 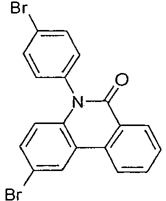 | 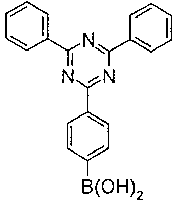 | 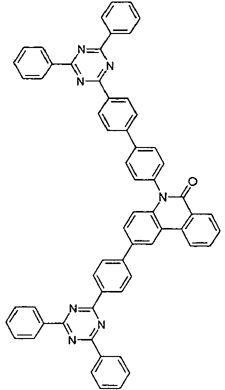 | 82% |
| | | 1313018-07-3 | | |

### Beispiel 15: 3,6-Dibrom-9,9-dimethyl-9,10-dihydro-acridin

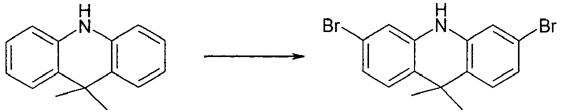

40 g (191 mmol) 9,9-Dimethyl-9,10-dihydro-acridin werden in 1000 mL CH₂Cl₂ vorgelegt. Anschließend tropft man unter Lichtausschluss bei -25 °C eine Lösung aus 68 g (382 mmol) NBS in 600 ml CH₂C₂l₂ hinzu, lässt auf Raumtemperatur kommen und rührt 4 h weiter bei dieser Temperatur. Anschließend wird die Mischung mit 150 mL Wasser versetzt und mit CH₂Cl₂ extrahiert. Die organische Phase wird über MgSO₄ getrocknet und die Lösungsmittel im Vakuum entfernt. Das Produkt wird mit Hexan heiß ausgerührt und abgesaugt. Ausbeute: 61.4 g (16 mmol), 70 % d. Th., Reinheit nach ¹H-NMR ca. 98 %.

### Beispiel 16: 9,9-Dimethyl-3,6-diphenyl-9,10-dihydro-acridin

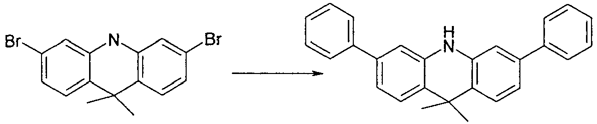

Ein entgaste Suspension von 52 g (143 mmol) 3,6-Dibrom-9,9-dimethyl-9,10-dihydro-acridin, 38.4 g (315 mmol) Phenylboronsäure und 60 g (286 mmol) Kaliumphosphat-hydrat in einem Gemisch aus 420 ml Dioxan, 840 ml Toluol und 420 ml Wasser wird unter gutem Rühren mit 1.7 g (5.7 mmol) Tri-o-tolylphosphin und dann mit 643 mg (2.8 mmol) Palladium(II)-acetat versetzt. Nach 5 h Erhitzen unter Rückfluss lässt man die Mischung erkalten. Der Niederschlag wird abgesaugt, dreimal mit 10 ml Ethanol / Wasser (1:1, v:v) und dreimal mit 5 ml Ethanol gewaschen, anschließend im Vakuum getrocknet und aus Dioxan umkristallisiert. Ausbeute: 41 g (113 mmol), 81 % d. Th., Reinheit nach ¹H-NMR ca. 99.9 %.

### Beispiel 17: 10-(2-Brombenzyl)-9,9-dimethyl-9,10-dihydro-acridin

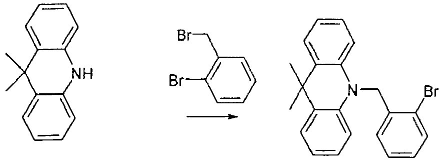

9.7 g (0.243 mol) NaH, 60%ig in Mineralöl, werden in 500 mL Dimethylformamid unter Schutzatmosphäre gelöst. 22.1 g (0.106 mol) 9,9-Dimethyl-9,10-dihydro-acridin werden in 500 mL DMF gelöst und zu der Reaktionsmischung zugetropft. Nach 1 h bei Raumtemperatur wird eine Lösung von 60.6 (242 mmol) 2-Brombenzylbromid in 500 mL DMF zugetropft. Das Reaktionsgemisch wird 1 h bei Raumtemperatur gerührt, dann auf Eis gegossen und dreimal mit Dichlormethan extrahiert. Die vereinigten organischen Phasen werden über Na₂SO₄ getrocknet und eingeengt. Der Rückstand wird mit Toluol heiß extrahiert und aus Toluol / n-Heptan umkristallisiert. Die Ausbeute beträgt 37 g (94 %).

Analog werden folgende Verbindungen erhalten:

| **Bsp**. | **Edukt 1** | **Edukt 2** | **Produkt** | **Ausbeute** |
|---|---|---|---|---|
| 17a | 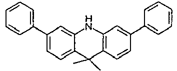 | 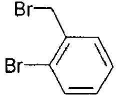 | 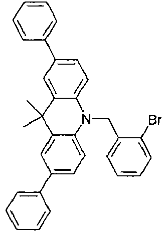 | 88% |
| 17b | 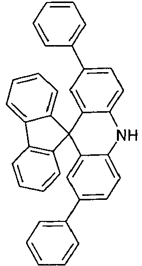 | 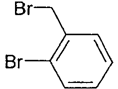 | 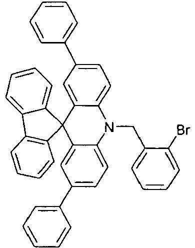 | 80% |
| | 1303969-38-1 | | | |

### Beispiel 18: 5-Biphenyl-4-yl-2-phenyl-5,6-dihydro-phenanthridin

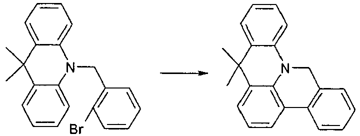

59.7 g (0.158 mol) 10-(2-Brombenzyl)-9,9-dimethyl-9,10-dihydro-acridin werden in 500 mL Dimethylformamid unter Schutzatmosphäre gelöst. Zu dieser Lösung werden 17.3 g (0.075 mol) Benzyltrimethylammoniumbromid und 31.28 g (0.226 mol) Kaliumcarbonat werden zugegeben. Anschließend wird unter Schutzgas 5.08 g (0.022 mol) Pd(OAc)₂ zugegeben, und das Gemisch wird bei 120 °C für 9 h gerührt. Nach dieser Zeit wird das Reaktionsgemisch auf Raumtemperatur gekühlt und mit Dichlormethan extrahiert. Die vereinigten organischen Phasen werden über Na₂SO₄ getrocknet und eingeengt. Der Rückstand wird aus n-Heptan umkristallisiert. Die Ausbeute beträgt 39.5 g (86 %).

Analog werden folgende Verbindungen erhalten:

| **Bsp**. | **Edukt 1** | **Produkt** | **Ausbeute** |
|---|---|---|---|
| 18a | 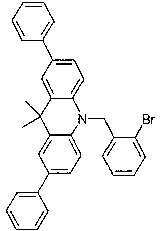 | 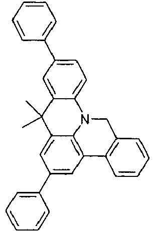 | 82% |
| 18b | 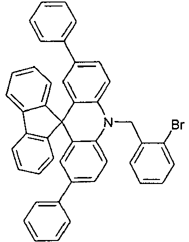 | 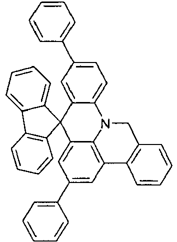 | 78% |

### Beispiel 19: 8,8-Dimethyl-8H-12b-aza-dibenzo[a,de]anthracen-13-on

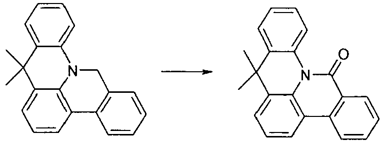

11.7 g (0.039 mol) 5-Biphenyl-4-yl-2-phenyl-5,6-dihydro-phenanthridin werden in 300 mL Dichlormethan gelöst. Zur diese Lösung werden 17.3 g (0.075 mol) Benzyltrimethylammoniumbromid und 62.13 g (0.393 mol) Kaliumpermanganat portionsweise zugegeben und zwei Tage bei Raumtemperatur gerührt. Nach dieser Zeit wird das restliche Kaliumpermanganat abfiltriert, die Lösung eingeengt und chromatographisch gereinigt (Laufmittel: Heptan/Dichloromethan, 5:1). Der Rückstand wird aus n-Heptan umkristallisiert. Die Ausbeute beträgt 9.8 g (80 %).

Analog wird die folgende Verbindung erhalten:

| **Bsp**. | **Edukt 1** | **Produkt** | **Ausbeute** |
|---|---|---|---|
| 19a | 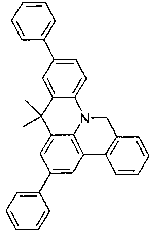 | 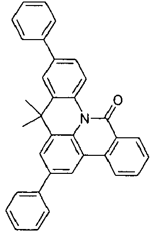 | 89% |

### Beispiel 20: 5-[3-(4,6-Diphenyl-[1,3,5]triazin-2-yl)-phenyl]-5H-phenanthridin-6-on

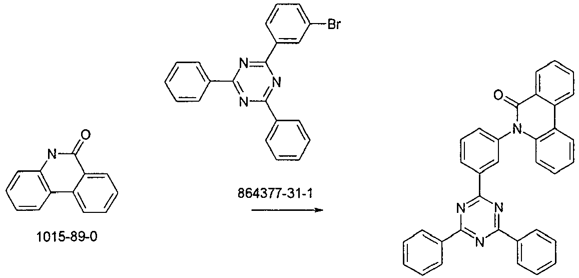

20 g (102,4 mmol) 5H-Phenanthridin-6-on, 43 g (112 mmol) 2-(3-Brom-phenyl)-4,6-diphenyl-[1,3,5]triazin, 2.3 g (10.2 mmol) 1,3-Bis[2-pyridyl]-1,3-propandion, 28.3 g (204 mmol) Kaliumcarbonat und 1.9 g (10.2) Kupferiodid werden in 1000 ml DMF unter Rückfluss 90 h gerührt. Die Lösung wird mit Wasser verdünnt und mit Essigester zweimal extrahiert, die vereinigten organischen Phasen über Na₂SO₄ getrocknet, einrotiert und chromatographisch (EtOAc/Hexan: 2/3) gereinigt. Der Rückstand wird aus Toluol und aus Dichlormethan umkristallisiert und abschließend im Hochvakuum sublimiert, Reinheit beträgt 99.9 %. Die Ausbeute beträgt 31 g (62 mmol), entsprechend 61 % der Theorie.

Analog werden folgende Verbindungen erhalten:

| **Bsp**. | **Edukt 1** | **Edukt 2** | **Produkt** | **Ausbeute** |
|---|---|---|---|---|
| 20a | 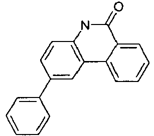 | 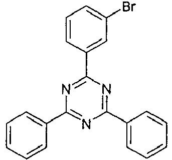 | 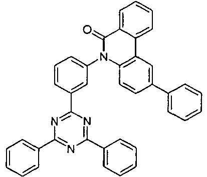 | 59% |
| | 157848-49-2 | | | |
| 20b | 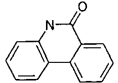 | 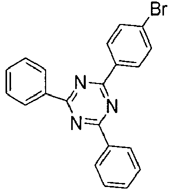 | 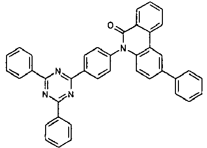 | 57% |
| | | 23449-08-3 | | |
| 20c | 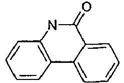 | 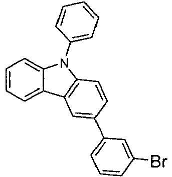 | 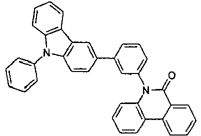 | 54% |
| | | 854952-59-3 | | |
| 20d | 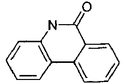 | 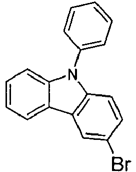 | 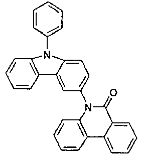 | 58% |
| | | 1153-85-1 | | |
| 20e | 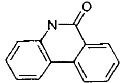 | 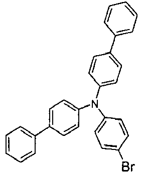 | 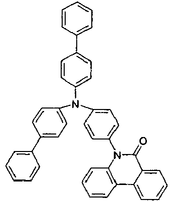 | |
| | | 1371655-72-9 | | |

### Herstellung der OLEDs

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, Materialien) angepasst wird.

In den folgenden Beispielen V1 bis E29 (siehe Tabellen 1 und 2) werden die Daten verschiedener OLEDs vorgestellt. Glasplättchen, die mit strukturiertem ITO (Indium-Zinn-Oxid) der Dicke 50 nm beschichtet sind werden zur verbesserten Prozessierung mit 20 nm PEDOT:PSS beschichtet (Poly(3,4-ethylendioxythiophen) poly(styrolsulfonat), bezogen als CLEVIOS™ P VP Al 4083 von Heraeus Precious Metals GmbH Deutschland, aus wässriger Lösung aufgeschleudert). Diese beschichteten Glasplättchen bilden die Substrate, auf welche die OLEDs aufgebracht werden. Die OLEDs haben prinzipiell folgenden Schichtaufbau: Substrat / optionale Lochinjektionsschicht (HIL) / Lochtransportschicht (HTL) / optionale Zwischenschicht (IL) / Elektronenblockerschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Eine Bezeichnung wie "3f" bezieht sich auf die Verbindung aus Beispiel 3f, etc. Die weiteren zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie 9h:VCbz1:TEG1 (60%:30%:10%) bedeutet hierbei, dass das Material 9h in einem Volumenanteil von 60%, VCbz1 in einem Anteil von 30% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m².

Die Daten der verschiedenen OLEDs sind in Tabelle 2 zusammengefasst. Die Beispiele V1 und V2 sind Vergleichsbeispiele gemäß dem Stand der Technik, die Beispiele E1-E19 zeigen Daten von OLEDs mit erfindungsgemäßen Materialien.

Im Folgenden werden einige der Beispiele näher erläutert, um die Vorteile der erfindungsgemäßen Verbindungen zu verdeutlichen. Es sei jedoch darauf hingewiesen, dass dies nur eine Auswahl der in Tabelle 2 gezeigten Daten darstellt.

### Verwendung von erfindungsgemäßen Verbindungen in der Elektronentransportschicht

Setzt man die Verbindung 9g in einer Elektronentransportschicht ein, so erhält man eine niedrige Betriebsspannung von 4.3 V und eine sehr gute Quanteneffizienz von etwas über 8%, bei Einsatz der Verbindung LaDi1 gemäß dem Stand der Technik sind diese Werte wesentlich schlechter (Beispiele V2 und E5).

Weiterhin erhält man sehr gute Lebensdauern mit erfindungsgemäßen Materialien: Für die OLED nach Beispiel E4 z. B. fällt die Leuchtdichte bei Betrieb mit 60 mA/cm² innerhalb von 210 h auf 70% ihres Anfangswertes ab.

### Verwendung von erfindungsgemäßen Verbindungen als Matrixmaterialien in phosphoreszierenden OLEDs

Bei Verwendung von erfindungsgemäßen Materialien als Einzelmatrixmaterial in Kombination mit dem grünen Dotanden TEG1 erhält man sehr niedrige Betriebsspannungen von 3.3 V und sehr gute externe Quanteneffizienzen bis zu 16.4% (Beispiele E10 und E11).

Auch in einem Mixed-Matrix System erhält man mit erfindungsgemäßen Materialien sehr gute Leistungsdaten. In Kombination mit dem Material VCbz1 z. B. erhält man über 17% externe Quanteneffizienz und benötigt eine sehr niedrige Betriebsspannung von 3.3 V (Beispiel E13).

Wählt man als zweite Komponente in einem Mixed-Matrix System eine Triazin oder Pyrimidin enthaltende Verbindung, so erhält man ebenfalls sehr gute Leistungsdaten, z.B. in der Kombination IC1 mit 19a eine externe Quanteneffzienz von über 16% (Beispiel E23).

Weiterhin erhält man sehr gute Lebensdauern: Für die OLED nach Beispiel E1 z. B. fällt die Leuchtdichte bei Betrieb mit 20 mA/cm² innerhalb von 170 h auf 70% ihres Anfangswertes ab. Für die OLED nach Beispiel E10 erhält man bei gleichen Bedingungen eine Lebensdauer von 190 h. Mit dem Material LaDi1 nach dem Stand der Technik erhält man dagegen bei gleichen Bedingungen nur eine Lebensdauer von 130 h (OLED nach Beispiel V1). In Mixed-Matrix Systemen lassen sich noch bessere Lebensdauern erzielen, bei Betrieb mit 20 mA/cm² erhält man für eine OLED nach Beispiel E16 z.B. einen Abfall auf 70% innerhalb von 330 h. Bei Betrieb einer OLED gemäß Beispiel E23 mit 20 mA/cm² erhält man einen Abfall auf 80% der Anfangsleuchtdichte in 305h, für Beispiel E22 sogar erst nach 340h.

**Tabelle 1: Aufbau der OLEDs**

| Bsp. | HIL Dicke | HTL Dicke | IL Dicke | EBL Dicke | EML Dicke | HBL Dicke | ETL Dicke | EIL Dicke |
|---|---|---|---|---|---|---|---|---|
| V1 | --- | SpA1 70nm | HATCN 5nm | SpMA1 90nm | LaDi1:TEG1 (90%:10%) 30nm | IC1 10nm | ST1:LiQ (50%:50%) 30nm | --- |
| V2 | HATCN 5nm | SpA1 140nm | HATCN 5nm | SpMA1 20nm | M1:D1 (95%:5%) 20nm | --- | LaDi1 30nm | LiQ 3nm |
| E1 | --- | SpA1 70nm | HATCN 5nm | SpMA1 90nm | 3:TEG1 (90%:10%) 30nm | IC1 10nm | ST4:LiQ (50%:50%) 30nm | --- |
| E2 | --- | SpA1 70nm | HATCN 5nm | SpMA1 90nm | 3e:TEG1 (90%:10%) 30nm | IC1 10nm | ST1:LiQ (50%:50%) 30nm | --- |
| E3 | --- | SpA1 70nm | HATCN 5nm | SpMA1 90nm | 3f:TEG1 (90%:10%) 30nm | IC1 10nm | ST1:LiQ (50%:50%) 30nm | --- |
| E4 | HATCN 5nm | SpA1 140nm | HATCN 5nm | SpMA1 20nm | M1:D1 (95%:5%) 20nm | --- | 9f:LiQ (50%:50%) 30nm | --- |
| E5 | HATCN 5nm | SpA1 140nm | HATCN 5nm | SpMA1 20nm | M1:D1 (95%:5%) 20nm | --- | 9g 30nm | LiQ 3nm |
| E6 | HATCN 5nm | SpA1 140nm | HATCN 5nm | SpMA1 20nm | M1:D1 (95%:5%) 20nm | --- | 3h 30nm | LiQ 3nm |
| E7 | --- | SpA1 70nm | HATCN 5nm | SpMA1 90nm | 3h:TEG1 (90%:10%) 30nm | IC1 10nm | ST1:LiQ (50%:50%) 30nm | --- |
| E8 | --- | SpA1 70nm | HATCN 5nm | SpMA1 90nm | 3j:IC1:TEG1 (60%:30%:10%) 30nm | IC1 10nm | ST1:LiQ (50%:50%) 30nm | --- |
| E9 | --- | SpA1 70nm | HATCN 5nm | SpMA1 90nm | 3k:TEG1 (90%:10%) 30nm | IC1 10nm | ST1:LiQ (50%:50%) 30nm | --- |
| E10 | --- | SpA1 70nm | HATCN 5nm | SpMA1 90nm | 8a:TEG1 (90%:10%) 30nm | IC1 10nm | ST1:LiQ(50%:50%) 30nm | --- |
| E11 | --- | SpA1 70nm | HATCN 5nm | SpMA1 90nm | 8:TEG1 (90%:10%) 30nm | IC1 10nm | ST1:LiQ (50%:50%) 30nm | --- |
| E12 | --- | SpA1 70nm | HATCN 5nm | SpMA1 90nm | 8b:TEG1 (90%:10%) 30nm | IC1 10nm | ST1:LiQ (50%:50%) 30nm | --- |
| E13 | --- | SpA1 70nm | HATCN 5nm | SpMA1 90nm | 9g:VCbz1:TEG1 (60%:30%:10%) 30nm | IC1 10nm | ST1:LiQ (50%:50%) 30nm | --- |
| E14 | --- | SpA1 90nm | HATCN 5nm | SpMA1 130nm | 9c:TER1 (92%:8%) 40nm | --- | ST1:LiQ (50%:50%) 40nm | --- |
| E15 | --- | SpA1 90nm | HATCN 5nm | SpMA1 130nm | 9d:TER1 (92%:8%) 40nm | --- | ST1:LiQ (50%:50%) 40nm | --- |
| E16 | --- | SpA1 70nm | HATCN 5nm | SpMA1 90nm | 9b:IC1:TEG1 (60%:30%) 30nm | ICl 10nm | ST1:LiQ (50%:50%) 30nm | --- |
| E17 | --- | SpA1 70nm | HATCN 5nm | SpMA1 90nm | IC1:TEG1 (90%:10%) 30nm | --- | 3a 40nm | LiQ 3nm |
| E18 | --- | SpA1 90nm | HATCN 5nm | SpMA1 130nm | 3c:TER1 (92%:8%) 40nm | --- | ST1:LiQ (50%:50%) 40nm | --- |
| E19 | --- | SpA1 70nm | HATCN 5nm | SpMA1 90nm | 3g:TEG1 (90%:10%) 30nm | IC1 10nm | ST1:LiQ (50%:50%) 30nm | --- |
| E20 | --- | SpA1 90nm | HATCN 5nm | SpMA1 130nm | IC1:TEG1 (90%:10%) 40nm | --- | 20:LiQ (50%:50%) 40nm | --- |
| E21 | --- | SpA1 90nm | HATCN 5nm | SpMA1 130nm | IC1:TEG1 (90%:10%) 40nm | --- | 20 40nm | LiF 1nm |
| E22 | --- | SpA1 70nm | HATCN 5nm | SpMA1 90nm | 3:IC2:TEG1 (60%:30%:10%) 40nm | --- | ST1:LiQ (50%:50%) 30nm | --- |
| E23 | --- | SpA1 70nm | HATCN 5nm | SpMA1 90nm | 19a:IC1:TEG1 (45%:45%:10%)30nm | IC1 10nm | ST1:LiQ (50%:50%) 30nm | --- |
| E24 | --- | SpA1 70nm | HATCN 5nm | SpMA1 90nm | 19a:IC3:TEG1 (45%:45%:10%)30nm | --- | ST1:LiQ (50%:50%) 40nm | --- |
| E25 | --- | SpA1 70nm | HATCN 5nm | SpMA1 90nm | 19a:IC4:TEG1 (45%:45%:10%)30nm | IC1 10nm | ST1:LiQ (50%:50%) 30nm | --- |
| E26 | --- | SpA1 90nm | HATCN 5nm | SpMA1 130nm | 19a:TEG1 (90%:10%) 40nm | --- | 19a 40nm | LiQ 3nm |
| E27 | --- | SpA1 70nm | HATCN 5nm | SpMA1 90nm | 19a:3e:TEG1 (45%:45%:10%)30nm | IC1 10nm | ST1:LiQ (50%:50%) 30nm | --- |
| E28 | --- | SpA1 70nm | HATCN 5nm | SpMA1 90nm | 8a:IC2:TEG1 (45%:45%:10%) 40nm | --- | ST1:LiQ (50%:50%) 30nm | --- |
| E29 | --- | SpA1 70nm | HATCN 5nm | SpMA1 90nm | 3:ST2:TEG1 (60%:30%:10%) 40nm | --- | ST1:LiQ (50%:50%) 30nm | --- |

**Tabelle 2: Daten der OLEDs**

| Bsp. | U1000 (V) | SE1000 (cd/A) | LE1000 (lm/W) | EQE 1000 | CIE x/y bei 1000 cd/m² |
|---|---|---|---|---|---|
| V1 | 4.5 | 46 | 33 | 13.0% | 0.33/0.62 |
| V2 | 5.3 | 6.6 | 3.9 | 6.1% | 0.14/0.13 |
| E1 | 4.4 | 48 | 35 | 13.6% | 0.34/0.62 |
| E2 | 4.5 | 48 | 33 | 13.3% | 0.34/0.62 |
| E3 | 4.0 | 51 | 41 | 14.3% | 0.34/0.63 |
| E4 | 4.4 | 8.2 | 5.8 | 7.6% | 0.14/0.13 |
| E5 | 4.3 | 8.7 | 6.4 | 8.1% | 0.14/0.13 |
| E6 | 4.6 | 7.1 | 4.8 | 6.6% | 0.14/0.13 |
| E7 | 3.7 | 55 | 48 | 15.5% | 0.33/0.62 |
| E8 | 3.7 | 55 | 46 | 15.2% | 0.34/0.63 |
| E9 | 4.1 | 52 | 40 | 14.4% | 0.33/0.62 |
| E10 | 3.4 | 59 | 54 | 16.4% | 0.33/0.63 |
| E11 | 3.3 | 58 | 55 | 16.2% | 0.34/0.62 |
| E12 | 3.4 | 56 | 52 | 15.8% | 0.34/0.62 |
| E13 | 3.3 | 63 | 60 | 17.4% | 0.33/0.62 |
| E14 | 4.3 | 11.8 | 8.7 | 12.8% | 0.67/0.33 |
| E15 | 4.4 | 10.8 | 7.7 | 11.8% | 0.67/0.33 |
| E16 | 3.6 | 54 | 47 | 15.0% | 0.33/0.63 |
| E17 | 3.7 | 57 | 49 | 15.9% | 0.32/0.62 |
| E18 | 4.7 | 10.1 | 6.8 | 11.1% | 0.67/0.33 |
| E19 | 4.5 | 46 | 32 | 12.8% | 0.34/0.62 |
| E20 | 3.3 | 64 | 61 | 17.3% | 0.34/0.62 |
| E21 | 3.8 | 60 | 50 | 16.0% | 0.34/0.62 |
| E22 | 3.8 | 59 | 49 | 15.8% | 0.34/0.63 |
| E23 | 3.8 | 60 | 50 | 16.3% | 0.35/0.62 |
| E24 | 3.7 | 61 | 52 | 16.7% | 0.34/0.62 |
| E25 | 3.5 | 55 | 49 | 15.4% | 0.34/0.63 |
| E26 | 4.4 | 55 | 39 | 15.2% | 0.33/0.62 |
| E27 | 3.6 | 57 | 48 | 15.7% | 0.32/0.62 |
| E28 | 3.2 | 58 | 56 | 16.8% | 0.33/0.63 |
| E29 | 4.2 | 61 | 46 | 16.8% | 0.33/0.63 |

**Tabelle 3: Strukturformeln der Materialien für die OLEDs**

| | |
|---|---|
| 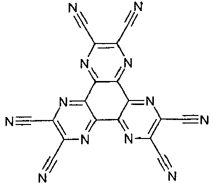 | 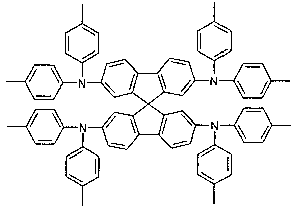 |
| HATCN | SpA1 |
| 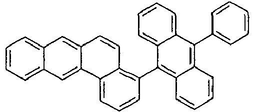 | 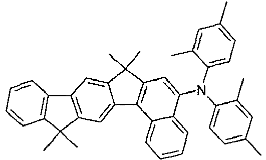 |
| M1 | D1 |
| 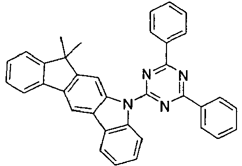 | 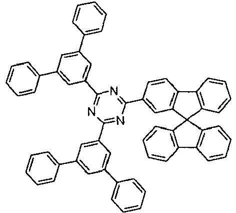 |
| IC1 | ST1 |
| 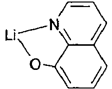 | 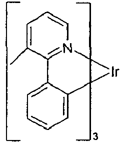 |
| LiQ | TEG1 |
| 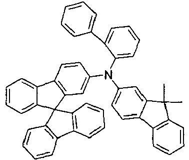 | 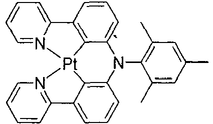 |
| SpMA¹ | TER1 |
| 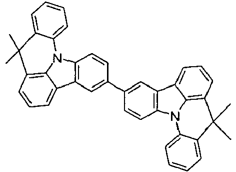 | 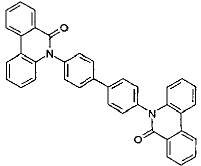 |
| VCbz1 | LaDi1 (Stand der Technik) |
| 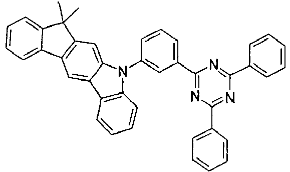 | 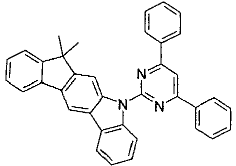 |
| IC2 | IC3 |
| 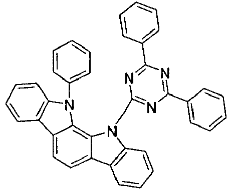 | 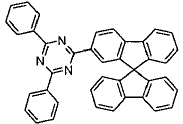 |
| IC4 | ST2 |

## Patentansprüche

1. Elektronische Vorrichtung enthaltend eine Verbindung gemäß Formel (1), 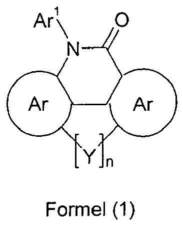 wobei für die verwendeten Symbole und Indizes gilt:
Ar ist bei jedem Auftreten gleich oder verschieden eine Aryl- oder Heteroarylgruppe mit 5 bis 13 aromatischen Ringatomen, welche durch einen oder mehrere Reste R substituiert sein kann;
Ar¹ ist ein aromatisches oder heteroaromatisches Ringsystem mit 5 bis 24 aromatischen Ringatomen, welches durch einen oder mehrere Reste R substituiert sein kann und welches keine Aryl- oder Heteroarylgruppen mit mehr als zwei direkt aneinander kondensierten aromatischen Sechsringen aufweist;
Y ist -C(=O)-N(Ar¹)-, -C(=O)-O-, -CR¹=CR¹-, -CR¹=N-, C(R¹)₂, NR¹, O, S, C(=O), C(=S), C(=NR¹), C(=C(R¹)₂), Si(R¹)₂, BR¹, PR¹, P(=O)R¹, SO oder SO₂;
R, R¹ ist bei jedem Auftreten gleich oder verschieden ausgewählt aus der Gruppe bestehend aus H, D, F, Cl Br, I, CN, NO₂, N(Ar²)₂, N(R²)₂, C(=O)Ar², C(=O)R², P(=O)(Ar²)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R² substituiert sein kann, wobei eine oder mehrere nicht-benachbarte CH₂-Gruppen durch R²C=CR², C≡C, Si(R²)₂, C=O, CS, C=NR², P(=O)(R²), SO, SO₂, NR², O, S oder CONR² ersetzt sein können und wobei ein oder mehrere H-Atome durch D, F, Ci, Br, I, CN oder NO₂ ersetzt sein können, einem aromatischen oder heteroaromatischen Ringsystem mit 5 bis 60 aromatischen Ringatomen, das jeweils mit einem oder mehreren Resten R² substituiert sein kann, einer Aryloxy- oder Heteroaryloxygruppe mit 5 bis 60 aromatischen Ringatomen, die mit einem oder mehreren Resten R² substituiert sein kann, oder einer Kombination dieser Systeme, wobei optional zwei oder mehr benachbarte Substituenten R bzw. zwei oder mehr benachbarte Substituenten R¹ ein monocyclisches oder polycyclisches, aliphatisches, aromatisches oder heteroaromatisches Ringsystem bilden können, das mit einem oder mehreren Resten R² substituiert sein kann und welches keine Aryl- oder Heteroarylgruppen mit mehr als zwei direkt aneinander kondensierten aromatischen Sechsringen aufweist; weiterhin kann auch ein Rest R an Ar¹ mit einem Rest R an Ar ein aliphatisches Ringsystem bilden; mit der Maßgabe, dass durch Ringbildung der Reste R bzw. R¹ keine Aryl- bzw. Heteroarylgruppe mit mehr als zwei direkt aneinander kondensierten Arylgruppen entsteht;
Ar² ist bei jedem Auftreten gleich oder verschieden ein aromatisches oder heteroaromatisches Ringsystem mit 5-30 aromatischen Ringatomen, das mit einem oder mehreren nicht-aromatischen Resten R² substituiert sein kann und welches keine Aryl- oder Heteroarylgruppen mit mehr als zwei direkt aneinander kondensierten aromatischen Sechsringen aufweist; dabei können zwei Reste Ar², welche an dasselbe N-Atom oder P-Atom binden, auch durch eine Einfachbindung oder eine Brücke, ausgewählt aus N(R²), C(R²)₂ oder O, miteinander verbrückt sein;
R² ist ausgewählt aus der Gruppe bestehend aus H, D, F, CN, einem aliphatischem Kohlenwasserstoffrest mit 1 bis 20 C-Atomen, einem aromatischem oder heteroaromatischem Ringsystem mit 5 bis 30 aromatischen Ringatomen, in dem ein oder mehrere H-Atome durch D, F, Cl, Br, I oder CN ersetzt sein können, wobei zwei oder mehr benachbarte Substituenten R² miteinander ein mono- oder polycyclisches, aliphatisches, aromatisches oder heteroaromatisches Ringsystem bilden können;
n ist bei jedem Auftreten gleich oder verschieden 0 oder 1, wobei n = 0 bedeutet, dass keine Gruppe Y vorhanden ist und statt dessen in den Positionen an Ar, in denen in Formel (1) Y gebunden ist, ein Substituent R gebunden ist oder ein Heteroatom der Gruppe Ar vorhanden ist;
mit der Maßgabe, dass die Reste an Ar oder Ar¹ keine Lactamgruppe enthalten.

2. Elektronische Vorrichtung nach Anspruch 1, ausgewählt aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen farbstoffsensibilisierten Solarzellen, organischen optischen Detektoren, organischen Photorezeptoren, organischen Feld-Quench-Devices, lichtemittierenden elektrochemischen Zellen, organischen Laserdioden (O-Laser) und "organic plasmon emitting devices".

3. Elektronische Vorrichtung nach Anspruch 1 oder 2, **dadurch gekennzeichnet, dass** Ar gleich oder verschieden bei jedem Auftreten ausgewählt ist aus einer Aryl- oder Heteroarylgruppe mit 6 aromatischen Ringatomen oder einer Heteroarylgruppe mit 5 aromatischen Ringatomen, welche jeweils mit einem oder mehreren Resten R substituiert sein kann, wobei zwei Substituenten R auch miteinander ein weiteres Ringsystem bilden können.

4. Elektronische Vorrichtung nach einem oder mehreren der Ansprüche 1 bis 3, **dadurch gekennzeichnet, dass** Ar für n = 0 ausgewählt ist aus den Gruppen der folgenden Formeln (2) bis (5) und für n = 1 aus den Gruppen der folgenden Formeln (6) bis (8), mit der Maßgabe, dass für n = 1 mindestens eine Gruppe Ar für eine Gruppe der Formel (6) steht, 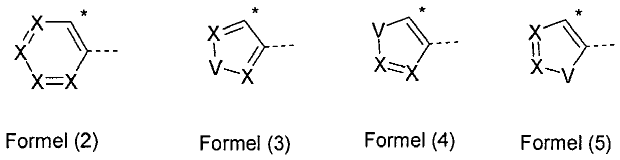 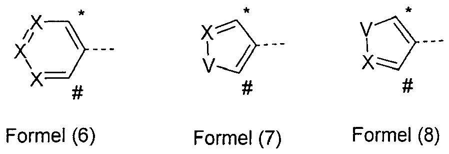 wobei die gestrichelte Bindung die Verknüpfung mit der zweiten Gruppe Ar andeutet, # die Position der Verknüpfung mit Y andeutet, * die Verknüpfung mit dem Stickstoffatom bzw. der Carbonylgruppe des Lactams andeutet und weiterhin gilt:
X ist gleich oder verschieden bei jedem Auftreten CR oder N; oder zwei benachbarte Gruppen X in Formel (2) oder Formel (6) stehen für eine Gruppe der folgenden Formel (9) oder (10), 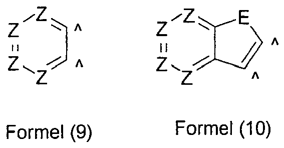 wobei E für NR, CR₂, O oder S steht, Z gleich oder verschieden bei jedem Auftreten für CR oder N steht und ^ die entsprechenden benachbarten Gruppen X in Formel (2) oder Formel (6) andeuten;
V ist NR, O oder S.

5. Elektronische Vorrichtung nach einem oder mehreren der Ansprüche 1 bis 4, ausgewählt aus den Verbindungen der Formeln (11) bis (21), 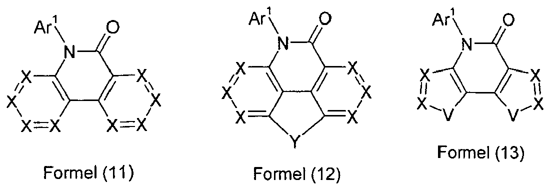 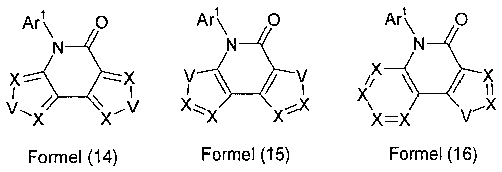 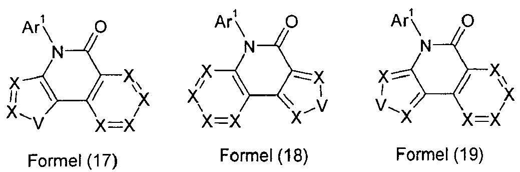 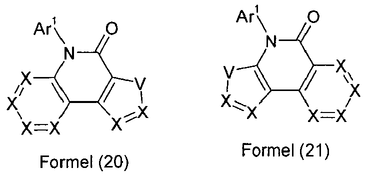 wobei die verwendeten Symbole die in Anspruch 1 und 4 genannten Bedeutungen aufweisen.

6. Elektronische Vorrichtung nach Anspruch 4 oder 5, **dadurch gekennzeichnet, dass** alle Gruppen X gleich oder verschieden bei jedem Auftreten für CR stehen.

7. Elektronische Vorrichtung nach einem oder mehreren der Ansprüche 1 bis 6, **dadurch gekennzeichnet, dass** die Verbindungen der Formel (1) ausgewählt sind aus den Verbindungen der Formeln (11 b), (11 c), (11d), (12b), (12c) und (12d), 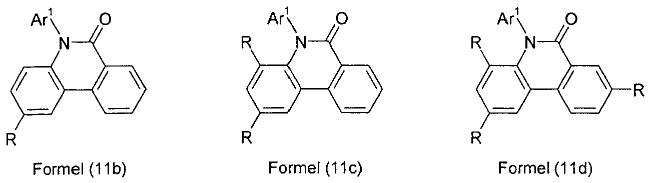 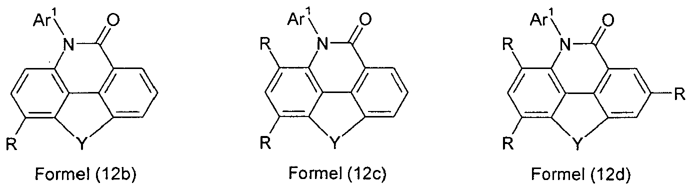 wobei die verwendeten Symbole die in Anspruch 1 genannten Bedeutungen aufweisen.

8. Elektronische Vorrichtung nach einem oder mehreren der Ansprüche 1 bis 7, **dadurch gekennzeichnet, dass** Ar¹ ausgewählt ist aus der Gruppe bestehend aus Benzol, ortho-, meta- oder para-Biphenyl, ortho-, meta-, para- oder verzweigtes Terphenyl, lineares oder verzweigtes Quaterphenyl, Fluoren, Spirobifluoren, Carbazol, Dibenzothiophen, Dibenzofuran, 1,3,5-Triazin, Pyridin, Pyrimidin, Indenocarbazol, verbrücktes Carbazol oder Indolocarbazol oder einer Kombination aus zwei oder drei dieser Gruppen, wobei diese Gruppen jeweils auch durch einen oder mehrere Reste R substituiert sein können.

9. Elektronische Vorrichtung nach einem oder mehreren der Ansprüche 1 bis 8, **dadurch gekennzeichnet, dass** R gleich oder verschieden bei jedem Auftreten ausgewählt ist aus der Gruppe bestehend aus H, D, F, CN, N(Ar²)₂, C(=O)Ar², einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten R² substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 30 aromatischen Ringatomen, das jeweils mit einem oder mehreren Resten R² substituiert sein kann, oder einer Aryloxy- oder Heteroaryloxygruppe mit 5 bis 30 aromatischen Ringatomen, die mit einem oder mehreren Resten R² substituiert sein kann.

10. Elektronische Vorrichtung nach einem oder mehreren der Ansprüche 1 bis 9, wobei es sich um eine organische Elektrolumineszenzvorrichtung handelt, **dadurch gekennzeichnet, dass** die Verbindung gemäß Formel (1) als Matrixmaterial für fluoreszierende oder phosphoreszierende Emitter und/oder in einer Lochblockierschicht und/oder in einer Elektronentransportschicht und/oder in einer elektronenblockierenden bzw. exzitonenblockierenden Schicht und/oder in einer Lochtransportschicht und/oder in einer optischen Auskopplungsschicht eingesetzt wird.

11. Verbindung gemäß Formel (1'), 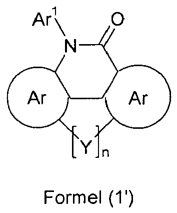 wobei für die verwendeten Symbole und Indizes gilt:
Ar ist bei jedem Auftreten gleich oder verschieden eine Aryl- oder Heteroarylgruppe mit 5 bis 13 aromatischen Ringatomen, welche durch einen oder mehrere Reste R substituiert sein kann;
Ar¹ ist ein aromatisches oder heteroaromatisches Ringsystem mit 5 bis 24 aromatischen Ringatomen, welches durch einen oder mehrere Reste R substituiert sein kann und welches keine Aryl- oder Heteroarylgruppen mit mehr als zwei direkt aneinander kondensierten aromatischen Sechsringen aufweist;
Y ist -C(=O)-N(Ar¹)-, -C(=O)-O-, -CR¹=CR¹-, -CR¹=N-, C(R¹)₂, NR¹, O, S, C(=O), C(=S), C(=NR¹), C(=C(R¹)₂), Si(R¹)₂, BR¹, PR¹, P(=O)R , SO oder SO₂;
R, R¹ ist bei jedem Auftreten gleich oder verschieden ausgewählt aus der Gruppe bestehend aus H, D, F, I, N(Ar²)₂, C(=O)Ar², C(=O)R², P(=O)(Ar²)₂, einer geradkettigen Alkylgruppe mit 1 bis 40 C-Atomen oder einer verzweigten oder cyclischen Alkylgruppe mit 3 bis 40 C-Atomen oder einer Alkenyl- oder Alkinylgruppe mit 2 bis 40 C-Atomen, die jeweils mit einem oder mehreren Resten R² substituiert sein kann, wobei eine oder mehrere nicht-benachbarte CH₂-Gruppen durch R²C=CR², C=C, Si(R²)₂, C=O, C=S, C=NR², P(=O)(R²), SO, SO₂, S oder CONR² ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R² substituiert sein kann, einer Aryloxy- oder Heteroaryloxygruppe mit 5 bis 60 aromatischen Ringatomen, die mit einem oder mehreren Resten R² substituiert sein kann, oder einer Kombination dieser Systeme, wobei optional zwei oder mehr benachbarte Substituenten R bzw. zwei oder mehr benachbarte Substituenten R¹ ein monocyclisches oder polycyclisches, aliphatisches, aromatisches oder heteroaromatisches Ringsystem bilden können, das mit einem oder mehreren Resten R² substituiert sein kann; weiterhin kann auch ein Rest R an Ar¹ mit einem Rest R an Ar ein aliphatisches Ringsystem bilden; mit der Maßgabe, dass durch Ringbildung der Reste R bzw. R¹ keine Aryl- bzw. Heteroarylgruppe mit mehr als zwei direkt aneinander kondensierten Arylgruppen entsteht;
Ar² ist bei jedem Auftreten gleich oder verschieden ein aromatisches oder heteroaromatisches Ringsystem mit 5-30 aromatischen Ringatomen, das mit einem oder mehreren nicht-aromatischen Resten R² substituiert sein kann; dabei können zwei Reste Ar², welche an dasselbe N-Atom oder P-Atom binden, auch durch eine Einfachbindung oder eine Brücke, ausgewählt aus N(R²), C(R²)₂ oder O, miteinander verbrückt sein;
R² ist ausgewählt aus der Gruppe bestehend aus H, D, F, einem aliphatischem Kohlenwasserstoffrest mit 1 bis 20 C-Atomen, einem aromatischem oder heteroaromatischem Ringsystem mit 5 bis 30 aromatischen Ringatomen, in dem ein oder mehrere H-Atome durch D, F, Cl, Br, I oder CN ersetzt sein können, wobei zwei oder mehr benachbarte Substituenten R³ miteinander ein mono- oder polycyclisches, aliphatisches, aromatisches oder heteroaromatisches Ringsystem bilden können;
n ist bei jedem Auftreten gleich oder verschieden 0 oder 1, wobei n = 1 bedeutet, dass keine Gruppe Y vorhanden ist und statt dessen in den Positionen an Ar, in denen in Formel (1) Y gebunden ist, ein Substituent R gebunden ist oder ein Heteroatom der Gruppe Ar vorhanden ist;
mit der Maßgabe, dass die Reste an Ar oder Ar¹ keine Lactamgruppe enthalten;
dabei sind die folgenden Verbindungen von der Erfindung ausgenommen:

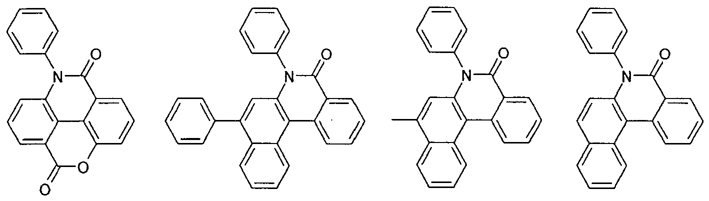

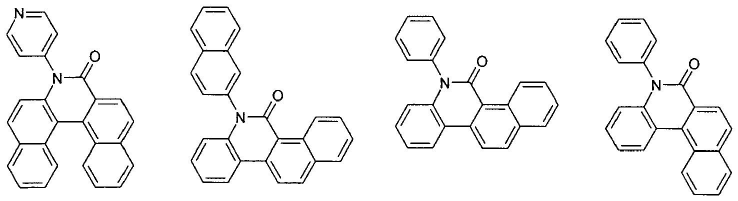

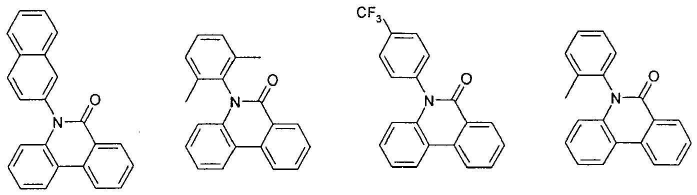

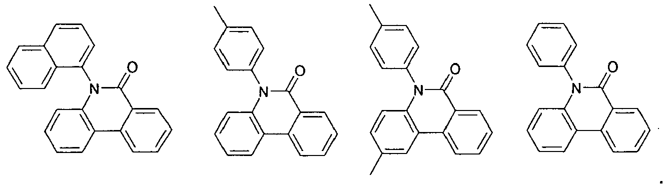

12. Verfahren zur Herstellung einer Verbindung gemäß Formel (1'), umfassend:
a) die Bindungsknüpfung zwischen dem Stickstoff eines Lactams und Ar¹; oder
b) die Bindungsknüpfung zwischen dem Stickstoff eines Amids und Ar; oder
c) die Bindungsknüpfung zwischen den beiden Gruppen Ar, die über eine Amidgruppe miteinander verknüpft sind; oder
d) die Bindungsknüpfung zwischen den beiden Gruppen Ar, die über eine Methylenamingruppe miteinander verknüpft sind, gefolgt von einer Oxidation zum entsprechenden Lactam.

13. Verwendung einer Verbindung nach Anspruch 11 in einer elektronischen Vorrichtung, insbesondere in einer organischen Elektrolumineszenzvorrichtung.

## Claims

1. Electronic device comprising a compound of the formula (1), 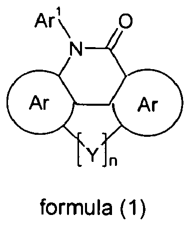 where the following applies to the symbols and indices used:
Ar is on each occurrence, identically or differently, an aryl or heteroaryl group having 5 to 13 aromatic ring atoms, which may be substituted by one or more radicals R;
Ar¹ is an aromatic or heteroaromatic ring system having 5 to 24 aromatic ring atoms, which may be substituted by one or more radicals R and which contains no aryl or heteroaryl groups having more than two aromatic six-membered rings condensed directly onto one another;
Y is -C(=O)-N(Ar¹)-, -C(=O)-O-, -CR¹=CR¹-, -CR¹=N-, C(R¹)₂, NR¹, O, S, C(=O), C(=S), C(=NR¹), C(=C(R¹)₂), Si(R¹)₂, BR¹, PR¹, P(=O)R¹, SO or SO₂;
R, R¹ is selected on each occurrence, identically or differently, from the group consisting of H, D, F, Cl, Br, I, CN, NO₂, N(Ar²)₂, N(R²)₂, C(=O)Ar², C(=O)R², P(=O)(Ar²)₂, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, each of which may be substituted by one or more radicals R², where one or more non-adjacent CH₂ groups may be replaced by R²C=CR², C=C, Si(R²)₂, C=O, C=S, C=NR², P(=O)(R²), SO, SO₂, NR², O, S or CONR² and where one or more H atoms may be replaced by D, F, Cl, Br, I, CN or NO₂, an aromatic or heteroaromatic ring system having 5 to 60 aromatic ring atoms, which may in each case be substituted by one or more radicals R², an aryloxy or heteroaryloxy group having 5 to 60 aromatic ring atoms, which may be substituted by one or more radicals R², or a combination of these systems, where two or more adjacent substituents R or two or more adjacent substituents R¹ may optionally form a monocyclic or polycyclic, aliphatic, aromatic or heteroaromatic ring system, which may be substituted by one or more radicals R² and which contains no aryl or heteroaryl groups having more than two aromatic six-membered rings condensed directly onto one another; a radical R on Ar¹ may furthermore also form an aliphatic ring system with a radical R on Ar; with the proviso that an aryl or heteroaryl group having more than two aryl groups condensed directly onto one another is not formed by ring formation of the radicals R or R¹;
Ar² is on each occurrence, identically or differently, an aromatic or heteroaromatic ring system having 5-30 aromatic ring atoms, which may be substituted by one or more non-aromatic radicals R² and which contains no aryl or heteroaryl groups having more than two aromatic six-membered rings condensed directly onto one another; two radicals Ar² which are bonded to the same N atom or P atom may also be bridged to one another here by a single bond or a bridge selected from N(R²), C(R²)₂ or O;
R² is selected from the group consisting of H, D, F, CN, an aliphatic hydrocarbon radical having 1 to 20 C atoms, an aromatic or heteroaromatic ring system having 5 to 30 aromatic ring atoms, in which one or more H atoms may be replaced by D, F, Cl, Br, I or CN, where two or more adjacent substituents R² may form a mono- or polycyclic, aliphatic, aromatic or heteroaromatic ring system with one another;
n is on each occurrence, identically or differently, 0 or 1, where n = 0 means that no group Y is present and instead a substituent R is bonded or a heteroatom of the group Ar is present in the positions on Ar in which Y is bonded in formula (1);
with the proviso that the radicals on Ar or Ar¹ do not contain a lactam group.

2. Electronic device according to Claim 1, selected from the group consisting of organic electroluminescent devices, organic integrated circuits, organic field-effect transistors, organic thin-film transistors, organic light-emitting transistors, organic solar cells, organic dye-sensitised solar cells, organic optical detectors, organic photoreceptors, organic field-quench devices, light-emitting electrochemical cells, organic laser diodes (O-lasers) and "organic plasmon emitting devices".

3. Electronic device according to Claim 1 or 2, **characterised in that** Ar is selected, identically or differently on each occurrence, from an aryl or heteroaryl group having 6 aromatic ring atoms or a heteroaryl group having 5 aromatic ring atoms, each of which may be substituted by one or more radicals R, where two substituents R may also form a further ring system with one another.

4. Electronic device according to one or more of Claims 1 to 3, **characterised in that** Ar for n = 0 is selected from the groups of the following formulae (2) to (5) and for n = 1 is selected from the groups of the following formulae (6) to (8), with the proviso that for n = 1, at least one group Ar stands for a group of the formula (6), 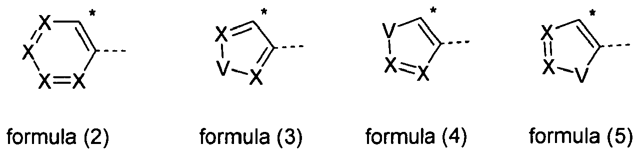 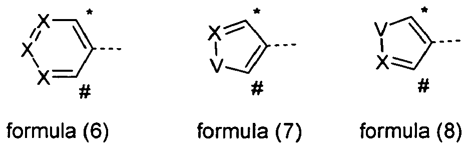 where the dashed bond indicates the link to the second group Ar, # indicates the position of the link to Y, * indicates the link to the nitrogen atom or the carbonyl group of the lactam and furthermore:
X is, identically or differently on each occurrence, CR or N; or two adjacent groups X in formula (2) or formula (6) stand for a group of the following formula (9) or (10), 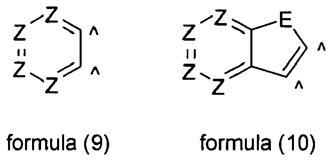 where E stands for NR, CR₂, O or S, Z stands, identically or differently on each occurrence, for CR or N and ^ indicate the corresponding adjacent groups X in formula (2) or formula (6);
V is NR, O or S.

5. Electronic device according to one or more of Claims 1 to 4, selected from the compounds of the formulae (11) to (21), 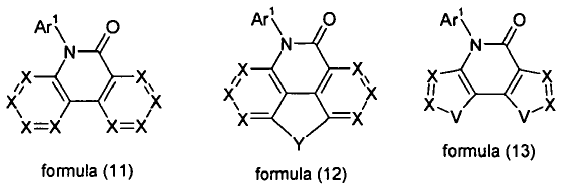 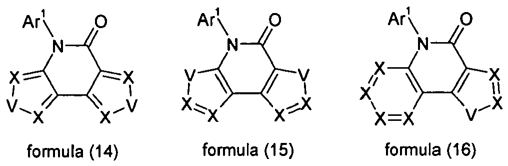 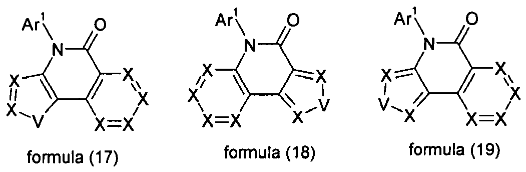 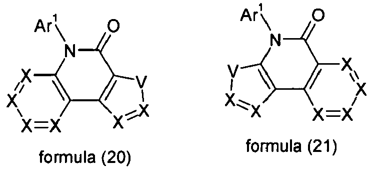 where the symbols used have the meanings given in Claim 1 and 4.

6. Electronic device according to Claim 4 or 5, **characterised in that** all groups X stand, identically or differently on each occurrence, for CR.

7. Electronic device according to one or more of Claims 1 to 6, **characterised in that** the compounds of the formula (1) are selected from the compounds of the formulae (11 b), (11 c), (11 d), (12b), (12c) and (12d), 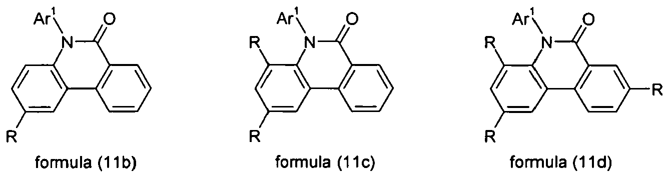 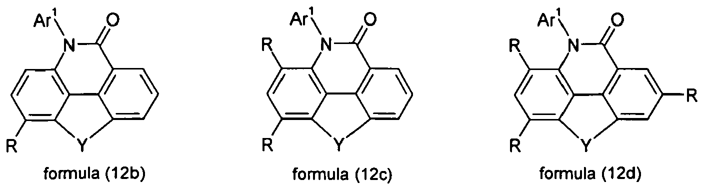 where the symbols used have the meanings given in Claim 1.

8. Electronic device according to one or more of Claims 1 to 7, **characterised in that** Ar¹ is selected from the group consisting of benzene, ortho-, meta- or para-biphenyl, ortho-, meta-, para- or branched terphenyl, linear or branched quaterphenyl, fluorene, spirobifluorene, carbazole, dibenzothiophene, dibenzofuran, 1,3,5-triazine, pyridine, pyrimidine, indenocarbazole, bridged carbazole or indolocarbazole or a combination of two or three of these groups, where these groups may each also be substituted by one or more radicals R.

9. Electronic device according to one or more of Claims 1 to 8, **characterised in that** R is selected, identically or differently on each occurrence, from the group consisting of H, D, F, CN, N(Ar²)₂, C(=O)Ar², a straight-chain alkyl or alkoxy group having 1 to 10 C atoms or a branched or cyclic alkyl or alkoxy group having 3 to 10 C atoms or an alkenyl group having 2 to 10 C atoms, each of which may be substituted by one or more radicals R², where one or more non-adjacent CH₂ groups may be replaced by O and where one or more H atoms may be replaced by D or F, an aromatic or heteroaromatic ring system having 5 to 30 aromatic ring atoms, which may in each case be substituted by one or more radicals R², or an aryloxy or heteroaryloxy group having 5 to 30 aromatic ring atoms, which may be substituted by one or more radicals R².

10. Electronic device according to one or more of Claims 1 to 9, which is an organic electroluminescent device, **characterised in that** the compound of the formula (1) is employed as matrix material for fluorescent or phosphorescent emitters and/or in a hole-blocking layer and/or in an electron-transport layer and/or in an electron-blocking or exciton-blocking layer and/or in a hole-transport layer and/or in an optical coupling-out layer.

11. Compound of the formula (1'), 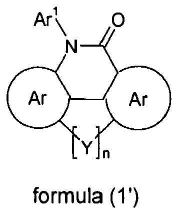 where the following applies to the symbols and indices used:
Ar is on each occurrence, identically or differently, an aryl or heteroaryl group having 5 to 13 aromatic ring atoms, which may be substituted by one or more radicals R;
Ar¹ is an aromatic or heteroaromatic ring system having 5 to 24 aromatic ring atoms, which may be substituted by one or more radicals R and which contains no aryl or heteroaryl groups having more than two aromatic six-membered rings condensed directly onto one another;
Y is -C(=O)-N(Ar¹)-, -C(=O)-O-, -CR¹=CR¹-, -CR¹=N-, C(R¹)₂, NR¹, O, S, C(=O), C(=S), C(=NR¹), C(=C(R¹)₂), Si(R¹)₂, BR¹, PR¹, P(=O)R¹, SO or SO₂;
R, R¹ is selected on each occurrence, identically or differently, from the group consisting of H, D, F, I, N(Ar²)₂, C(=O)Ar², C(=O)R², P(=O)(Ar²)₂, a straight-chain alkyl group having 1 to 40 C atoms or a branched or cyclic alkyl group having 3 to 40 C atoms or an alkenyl or alkynyl group having 2 to 40 C atoms, each of which may be substituted by one or more radicals R², where one or more non-adjacent CH₂ groups may be replaced by R²C=CR², C≡C, Si(R²)₂, C=O, C=S, C=NR², P(=O)(R²), SO, SO₂, S or CONR² and where one or more H atoms may be replaced by D, F, Cl, Br, I, CN or NO₂, an aromatic or heteroaromatic ring system having 5 to 60 aromatic ring atoms, which may in each case be substituted by one or more radicals R², an aryloxy or heteroaryloxy group having 5 to 60 aromatic ring atoms, which may be substituted by one or more radicals R², or a combination of these systems, where two or more adjacent substituents R or two or more adjacent substituents R¹ may optionally form a monocyclic or polycyclic, aliphatic, aromatic or heteroaromatic ring system, which may be substituted by one or more radicals R²; a radical R on Ar¹ may furthermore also form an aliphatic ring system with a radical R on Ar; with the proviso that an aryl or heteroaryl group having more than two aryl groups condensed directly onto one another is not formed by ring formation of the radicals R or R¹;
Ar² is on each occurrence, identically or differently, an aromatic or heteroaromatic ring system having 5-30 aromatic ring atoms, which may be substituted by one or more non-aromatic radicals R²; two radicals Ar² which are bonded to the same N atom or P atom may also be bridged to one another here by a single bond or a bridge selected from N(R²), C(R²)₂ or O;
R² is selected from the group consisting of H, D, F, an aliphatic hydrocarbon radical having 1 to 20 C atoms, an aromatic or heteroaromatic ring system having 5 to 30 aromatic ring atoms, in which one or more H atoms may be replaced by D, F, Cl, Br, I or CN, where two or more adjacent substituents R³ may form a mono- or polycyclic, aliphatic, aromatic or heteroaromatic ring system with one another;
n is on each occurrence, identically or differently, 0 or 1, where n = 1 means that no group Y is present and instead a substituent R is bonded or a heteroatom of the group Ar is present in the positions on Ar in which Y is bonded in formula (1);
with the proviso that the radicals on Ar or Ar¹ do not contain a lactam group;
the following compounds are excluded from the invention:

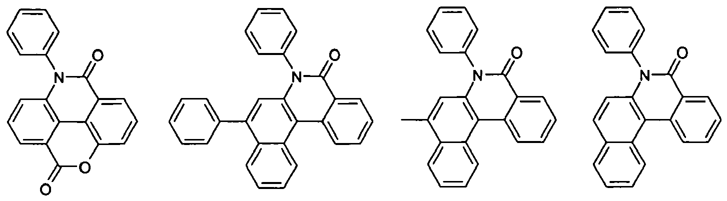

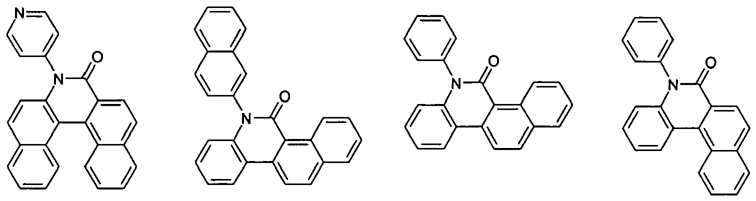

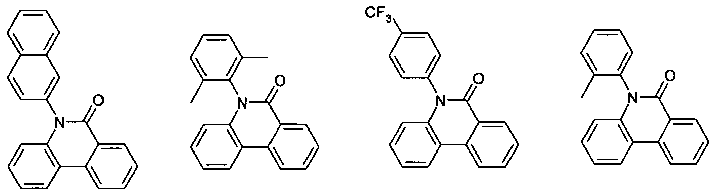

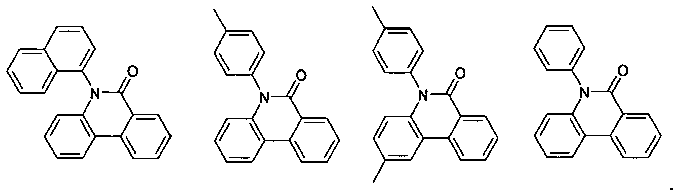

12. Process for the preparation of a compound of the formula (1'), comprising:
a) bond formation between the nitrogen of a lactam and Ar¹; or
b) bond formation between the nitrogen of an amide and Ar; or
c) bond formation between the two groups Ar which are linked to one another via an amide group; or
d) bond formation between the two groups Ar which are linked to one another via a methyleneamine group, followed by an oxidation to the corresponding lactam.

13. Use of a compound according to Claim 11 in an electronic device, in particular in an organic electroluminescent device.

## Revendications

1. Dispositif électronique comprenant un composé de la formule (1) : 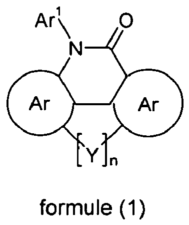 dans laquelle ce qui suit s'applique aux symboles et indices utilisés :
Ar est, pour chaque occurrence, de manière identique ou différente, un groupe aryle ou hétéroaryle comportant 5 à 13 atomes de cycle aromatique, lequel peut être substitué par un radical ou plusieurs radicaux R ;
Ar¹ est un système de cycle aromatique ou hétéroaromatique comportant 5 à 24 atomes de cycle aromatique, lequel peut être substitué par un radical ou plusieurs radicaux R et lequel ne contient pas de groupes aryle ou hétéroaryle comportant plus de deux cycles à six éléments condensés directement les uns sur les autres ;
Y est -C(=O)-N(Ar¹)-, -C(=O)-O-, -CR¹=CR¹-, -CR¹=N-, C(R¹)₂, NR¹, O, S, C(=O), C(=S), C(=NR¹), C(=C(R¹)₂), Si(R¹)₂, BR¹, PR¹, P(=O)R¹, SO ou SO_{2;}
R, R¹ est choisi, pour chaque occurrence, de manière identique ou différente, parmi le groupe constitué par H, D, F, Cl, Br, I, CN, NO₂, N(Ar²)₂, N(R²)₂, C(=O)Ar², C(=O)R², P(=O)(Ar²)₂, un groupe alkyle, alcoxy ou thioalkyle en chaîne droite comportant 1 à 40 atome(s) de C ou un groupe alkyle, alcoxy ou thioalkyle ramifié ou cyclique comportant 3 à 40 atomes de C ou un groupe alkényle ou alkynyle comportant 2 à 40 atomes de C, dont chacun peut être substitué par un radical ou plusieurs radicaux R², où un ou plusieurs groupe(s) CH₂ non adjacents peut/peuvent être remplacé(s) par R²C=CR², C≡C, Si(R²)₂, C=O, C=S, C=NR², P(=O)(R²), SO, SO₂, NR², O, S ou CONR² et où un ou plusieurs atome(s) de H peut/ peuvent être remplacé(s) par D, F, Cl, Br, I, CN ou NO₂, un système de cycle aromatique ou hétéroaromatique comportant 5 à 60 atomes de cycle aromatique, lequel peut dans chaque cas être substitué par un radical ou plusieurs radicaux R², un groupe aryloxy ou hétéroaryloxy comportant 5 à 60 atomes de cycle aromatique, lequel peut être substitué par un radical ou plusieurs radicaux R², ou une combinaison de ces systèmes, où deux substituants R adjacents ou plus ou deux substituants R¹ adjacents ou plus peuvent en option former un système de cycle aliphatique, aromatique ou hétéroaromatique monocyclique ou polycyclique, lequel peut être substitué par un radical ou plusieurs radicaux R² et lequel ne contient pas de groupes aryle ou hétéroaryle comportant plus de deux cycles aromatiques à six éléments condensés directement les uns sur les autres ; un radical R sur Ar¹ peut en outre également former un système de cycle aliphatique avec un radical R sur Ar ; étant entendu qu'un groupe aryle ou hétéroaryle comportant plus de deux groupes aryle condensés directement les uns sur les autres n'est pas formé par une formation de cycle des radicaux R ou R¹ ;
Ar² est, pour chaque occurrence, de manière identique ou différente, un système de cycle aromatique ou hétéroaromatique comportant 5-30 atomes de cycle aromatique, lequel peut être substitué par un radical ou plusieurs radicaux non aromatiques R² et lequel ne contient pas de groupes aryle ou hétéroaryle comportant plus de deux cycles aromatiques à six éléments condensés directement les uns sur les autres ; deux radicaux Ar² qui sont liés au même atome de N ou de P peuvent également être pontés l'un à l'autre ici par une liaison simple ou un pont choisi parmi N(R²), C(R²)₂ ou O ;
R² est choisi parmi le groupe constitué par H, D, F, CN, un radical hydrocarbone aliphatique comportant 1 à 20 atome(s) de C, un système de cycle aromatique ou hétéroaromatique comportant 5 à 30 atomes de cycle aromatique, où un ou plusieurs atome(s) de H peut/peuvent être remplacé(s) par D, F, Cl, Br, I ou CN, où deux substituants R² adjacents ou plus peuvent former un système de cycle aliphatique, aromatique ou hétéroaromatique monocyclique ou polycyclique l'un avec l'autre ou les uns avec les autres ;
n est, pour chaque occurrence, de manière identique ou différente, 0 ou 1, où n = 0 signifie qu'aucun groupe Y n'est présent et en lieu et place, un substituant R est lié ou un hétéroatome du groupe Ar est présent aux positions sur Ar au niveau desquelles Y est lié dans la formule (1) ;
étant entendu que les radicaux sur Ar ou Ar¹ ne contiennent pas un groupe lactame.

2. Dispositif électronique selon la revendication 1, choisi parmi le groupe constitué par des dispositifs électroluminescents organiques, des circuits intégrés organiques, des transistors à effet de champ organiques, des transistors à film mince organiques, des transistors à émission de lumière organiques, des cellules solaires organiques, des cellules solaires sensibilisées par colorant organiques, des détecteurs optiques organiques, des photorécepteurs organiques, des dispositifs à extinction de champ organiques, des cellules électrochimiques à émission de lumière, des diodes laser organiques (O-laser) et des "dispositifs à émission de plasmon organiques".

3. Dispositif électronique selon la revendication 1 ou 2, **caractérisé en ce que** Ar est choisi, de manière identique ou différente pour chaque occurrence, parmi un groupe aryle ou hétéroaryle comportant 6 atomes de cycle aromatique ou un groupe hétéroaryle comportant 5 atomes de cycle aromatique, dont chacun peut être substitué par un radical ou plusieurs radicaux R, où deux substituants R peuvent également former un autre système de cycle l'un avec l'autre.

4. Dispositif électronique selon une ou plusieurs des revendications 1 à 3, **caractérisé en ce que** Ar pour n = 0 est choisi parmi les groupes des formules qui suivent (2) à (5) et pour n = 1, est choisi parmi les groupes des formules qui suivent (6) à (8), étant entendu que pour n = 1, au moins un groupe Ar représente un groupe de la formule (6) : 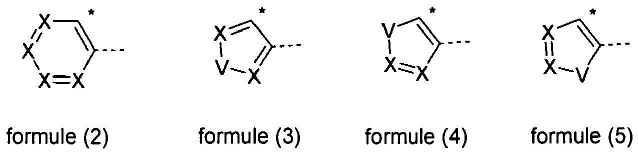 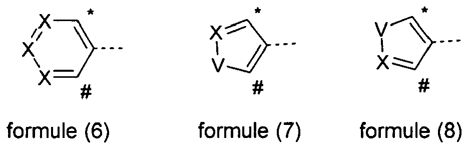 où le lien en pointillés indique la liaison sur le second groupe Ar, # indique la position de la liaison sur Y, * indique la liaison sur l'atome d'azote ou le groupe carbonyle du lactame et en outre :
X est, de manière identique ou différente pour chaque occurrence, CR ou N ; ou deux groupes adjacents X dans la formule (2) ou la formule (6) représentent un groupe de la formule (9) ou (10) qui suit : 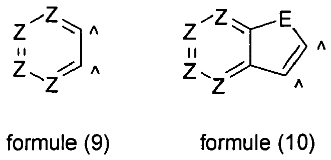 dans lesquelles E représente NR, CR₂, O ou S, Z représente, de manière identique ou différente pour chaque occurrence, CR ou N et ^ indique les groupes adjacents correspondants X dans la formule (2) ou la formule (6) ;
V est NR, O ou S.

5. Dispositif électronique selon une ou plusieurs des revendications 1 à 4, choisi parmi les composés des formules (11) à (21) : 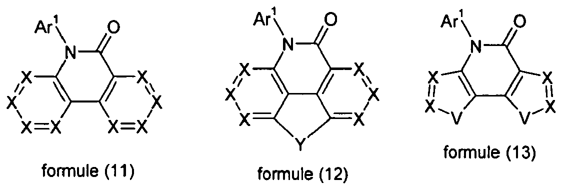 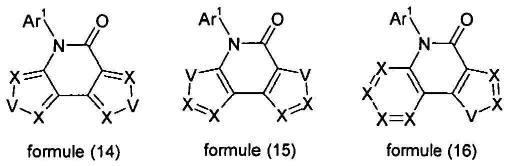 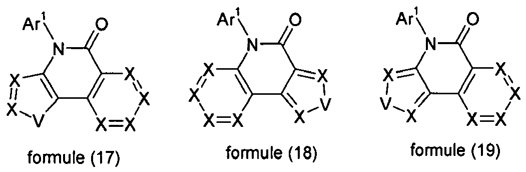 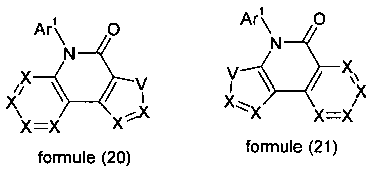 où les symboles utilisés présentent les significations données selon les revendications 1 et 4.

6. Dispositif électronique selon la revendication 4 ou 5, **caractérisé en ce que** tous les groupes X représentent, de manière identique ou différente pour chaque occurrence, CR.

7. Dispositif électronique selon une ou plusieurs des revendications 1 à 6, **caractérisé en ce que** les composés de la formule (1) sont choisis parmi les composés des formules (11b), (11c), (11d), (12b), (12c) et (12d) : 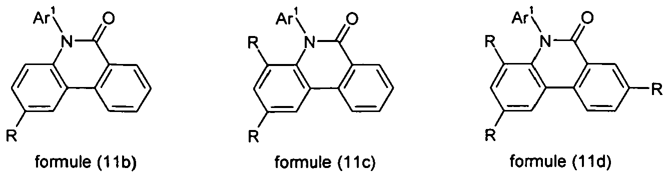 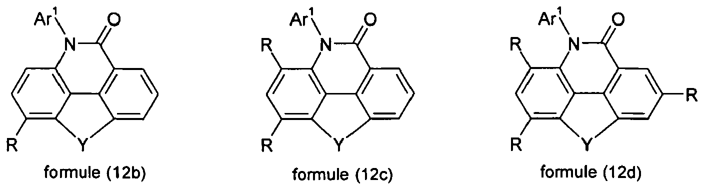 dans lesquelles les symboles utilisés présentent les significations données selon la revendication 1.

8. Dispositif électronique selon une ou plusieurs des revendications 1 à 7, **caractérisé en ce que** Ar¹ est choisi parmi le groupe constitué par benzène, ortho-, méta- ou para-biphényle, ortho-, méta-, para-terphényle ou terphényle ramifié, quaterphényle linéaire ou ramifié, fluorène, spirobifluorène, carbazole, dibenzothiophène, dibenzofurane, 1,3,5-triazine, pyridine, pyrimidine, indénocarbazole, carbazole ponté ou indolocarbazole ou une combinaison de deux ou trois de ces groupes, où ces groupes peuvent chacun également être substitués par un radical ou plusieurs radicaux R.

9. Dispositif électronique selon une ou plusieurs des revendications 1 à 8, **caractérisé en ce que** R est choisi, de manière identique ou différente pour chaque occurrence, parmi le groupe constitué par H, D, F, CN, N(Ar²)₂, C(=O)Ar², un groupe alkyle ou alcoxy en chaîne droite comportant 1 à 10 atome(s) de C ou un groupe alkyle ou alcoxy ramifié ou cyclique comportant 3 à 10 atomes de C ou un groupe alkényle comportant 2 à 10 atomes de C, dont chacun peut être substitué par un radical ou plusieurs radicaux R², où un ou plusieurs groupes CH₂ non adjacents peut/peuvent être remplacé(s) par O et où un ou plusieurs atome(s) de H peut/peuvent être remplacé(s) par D ou F, un système de cycle aromatique ou hétéroaromatique comportant 5 à 30 atomes de cycle aromatique, lequel peut dans chaque cas être substitué par un radical ou plusieurs radicaux R², ou un groupe aryloxy ou hétéroaryloxy comportant 5 à 30 atomes de cycle aromatique, lequel peut être substitué par un radical ou plusieurs radicaux R².

10. Dispositif électronique selon une ou plusieurs des revendications 1 à 9, lequel est un dispositif électroluminescent organique, **caractérisé en ce que** le composé de la formule (1) est utilisé en tant que matériau de matrice pour des émetteurs fluorescents ou phosphorescents et/ou dans une couche de blocage de trous et/ou dans une couche de transport d'électrons et/ou dans une couche de blocage d'électrons ou de blocage d'excitons et/ou dans une couche de transport de trous et/ou dans une couche de couplage en sortie optique.

11. Composé de la formule (1') : 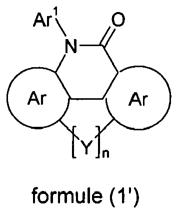 dans laquelle ce qui suit s'applique aux symboles et indices utilisés :
Ar est, pour chaque occurrence, de manière identique ou différente, un groupe aryle ou hétéroaryle comportant 5 à 13 atomes de cycle aromatique, lequel peut être substitué par un radical ou plusieurs radicaux R ;
Ar¹ est un système de cycle aromatique ou hétéroaromatique comportant 5 à 24 atomes de cycle aromatique, lequel peut être substitué par un radical ou plusieurs radicaux R et lequel ne contient pas de groupes aryle ou hétéroaryle comportant plus de deux cycles aromatique à six éléments condensés directement les uns sur les autres ;
Y est -C(=O)-N(Ar¹)-, -C(=O)-O-, -CR¹=CR¹-, -CR¹=N-, C(R¹)₂, NR¹, O, S, C(=O), C(=S), C(=NR¹), C(=C(R¹)₂), Si(R¹)₂, BR¹, PR¹, P(=O)R¹, SO ou SO₂;
R, R¹ est choisi, pour chaque occurrence, de manière identique ou différente, parmi le groupe constitué par H, D, F, I, N(Ar²)₂, C(=O)Ar², C(=O)R², P(=O)(Ar²)₂, un groupe alkyle en chaîne droite comportant 1 à 40 atome(s) de C ou un groupe alkyle ramifié ou cyclique comportant 3 à 40 atomes de C ou un groupe alkényle ou alkynyle comportant 2 à 40 atomes de C, dont chacun peut être substitué par un radical ou plusieurs radicaux R², où un ou plusieurs groupe(s) CH₂ non adjacents peut/peuvent être remplacé(s) par R²C=CR², C≡C, Si(R²)₂, C=O, C=S, C=NR², P(=O)(R²), SO, SO₂, S ou CONR² et où un ou plusieurs atome(s) de H peut/ peuvent être remplacé(s) par D, F, Cl, Br, I, CN ou NO₂, un système de cycle aromatique ou hétéroaromatique comportant 5 à 60 atomes de cycle aromatique, lequel peut dans chaque cas être substitué par un radical ou plusieurs radicaux R², un groupe aryloxy ou hétéroaryloxy comportant 5 à 60 atomes de cycle aromatique, lequel peut être substitué par un radical ou plusieurs radicaux R², ou une combinaison de ces systèmes, où deux substituants R adjacents ou plus ou deux substituants R¹ adjacents ou plus peuvent en option former un système de cycle aliphatique, aromatique ou hétéroaromatique monocyclique ou polycyclique, lequel peut être substitué par un radical ou plusieurs radicaux R^{2;} un radical R sur Ar¹ peut en outre également former un système de cycle aliphatique avec un radical R sur Ar ; étant entendu qu'un groupe aryle ou hétéroaryle comportant plus de deux groupes aryle condensés directement les uns sur les autres n'est pas formé par formation de cycle des radicaux R ou R^{1 ;}
Ar² est, pour chaque occurrence, de manière identique ou différente, un système de cycle aromatique ou hétéroaromatique comportant 5-30 atomes de cycle aromatique, lequel peut être substitué par un radical ou plusieurs radicaux non aromatiques R^{2 ;} deux radicaux Ar² qui sont liés au même atome de N ou au même atome de P peuvent également être pontés l'un à l'autre ici au moyen d'une liaison simple ou d'un pont choisi parmi N(R²), C(R²)₂ ou O ;
R² est choisi parmi le groupe constitué par H, D, F, un radical hydrocarbone aliphatique comportant 1 à 20 atome(s) de C, un système de cycle aromatique ou hétéroaromatique comportant 5 à 30 atomes de cycle aromatique, où un ou plusieurs atome(s) de H peut/peuvent être remplacé(s) par D, F, Cl, Br, I ou CN, où deux substituants R³ adjacents ou plus peuvent former un système de cycle aliphatique, aromatique ou hétéroaromatique monocyclique ou polycyclique l'un avec l'autre ou les uns avec les autres ;
n est, pour chaque occurrence, de manière identique ou différente, 0 ou 1, où n = 1 signifie qu'aucun groupe Y n'est présent et en lieu et place, un substituant R est lié ou un hétéroatome du groupe Ar est présent au niveau des positions sur Ar au niveau desquelles Y est lié dans la formule (1) ;
étant entendu que les radicaux sur Ar ou Ar¹ ne contiennent pas de groupe lactame ;
les composés qui suivent sont exclus de l'invention :

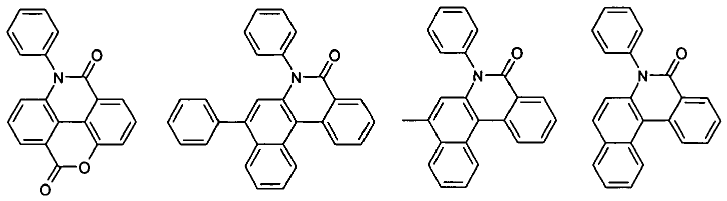

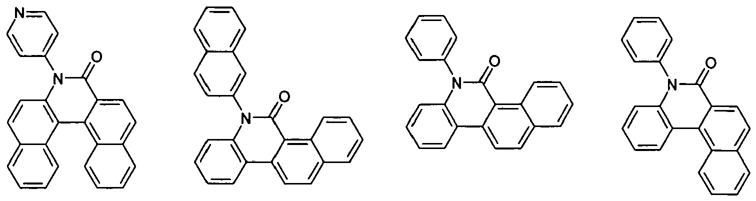

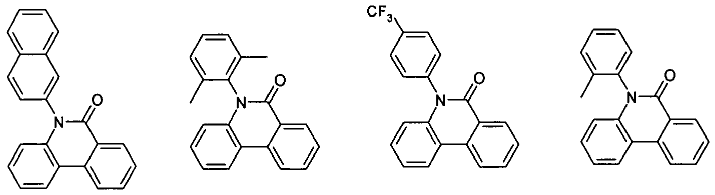

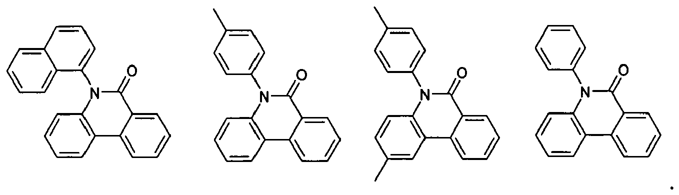

12. Procédé pour la préparation d'un composé de la formule (1'), comprenant :
a) une formation de liaison entre l'azote d'un lactame et Ar^{1 ;} ou
b) une formation de liaison entre l'azote d'un amide et Ar ; ou
c) une formation de liaison entre les deux groupes Ar qui sont liés l'un à l'autre via un groupe amide ; ou
d) une formation de liaison entre les deux groupes Ar qui sont liés l'un à l'autre via un groupe méthylèneamine, suivie par une oxydation sur le lactame correspondant.

13. Utilisation d'un composé selon la revendication 11 dans un dispositif électronique, en particulier dans un dispositif électroluminescent organique.
